# EUROPEAN PATENT APPLICATION

(11) **EP 1 627 923 A1**
(43) Date of publication of application: **22.02.2006**
(21) Application number: 04077343.4
(22) Date of filing: 18.08.2004
(51) Int. Cl.: C12Q 1/68

(54) **Means and methods for detecting and/or staging a follicular lymphoma cells**

(71) Applicant: Het Nederlands Kanker Instituut, 1066 CX Amsterdam (NL)
(72) Inventor: de Jong, Daphne, 2012 LR Haarlem (NL); Glas, Annuska, Maria, 1911 PX Uitgeest (NL); Wessels, Lodewyk, Frederik, Ary, 2614 XC Delft (NL)
(74) Representative: Winckels, Johannes Hubertus F.

(57) **Abstract**

The invention provides a method for detecting and/or staging a malignant functional cell comprising measuring an expression profile of said malignant cell and a non-malignant cell. Preferably, said non-malignant cell and said malignant cell are capable of interacting. In one aspect the invention provides reporter genes whose expression patterns are indicative for the presence and/or stage of follicular lymphoma.

### Annex to the application documents - subsequently filed sequences listing

SEQUENCE LISTING

<110>  Het Nederlands Kanker Instituut

<120>  Means and methods for detecting and/or staging a malignant functional
cell

<130>  P70258EP00

<140>  EP 04077343.4
<141>  2004-08-18

<160>  7

<170>  PatentIn version 3.1

<210>  1
<211>  64
<212>  DNA
<213>  Artificial Sequence

<220>
<223>  Linamp oligo

<400>  1
ggccagtgaa ttgtaatacg actcactata gggaggcggt tttttttttt tttttttttt      60

tttt                                                                    64


<210>  2
<211>  422
<212>  DNA
<213>  Artificial

<220>
<221>  misc_feature
<222>  (393)..(393)
<223>  "n" stands for unknown nucleic acid


<220>
<221>  misc_feature
<222>  (405)..(405)
<223>  "n" stands for unknown nucleic acid


<220>
<221>  misc_feature
<222>  (410)..(410)
<223>  "n" stands for unknown nucleic acid


<400>  2
ggtattttac attttatttt gttttttaga caaatgttca ttgtgactca cagaattgat      60

ttcatgcctc actaatgggt ctcacatata ccatgaaaaa tactgatata gagacatttt     120

gtgcctttgc tgtgttggta ccttgagtat gtctgcttga tgatgaaaga gactggctga     180

tctgctagac tgttgacttg tataagaaca tcttgaagtg gtgggaattt atcttagatt     240

ttcattcctg gatcattttt atggtctgat ccagtgagga gccaatgagg ttttccatgg     300

ctaggtcaaa tatcaggttg gagttgatgg gatattttta tagaactttc cccggaatgc     360

aacccttgac ctccagcccc agccgcagcg aanccgggcc ggggncccan cgctgcaagc     420

ct                                                                    422

<210>  3
<211>  501
<212>  DNA
<213>  Artificial Sequence

<220>
<223>  EST 7650

<400>  3
cacaaaataa aaatcactaa aaattcccac aaatcttgtt tctggcactt tagaaaaact      60

gcaaaaaaat acgtaataaa gaatacatat atatatatct acacacaaat tatatatcta     120

tctatctata cagcggaacc acaagagaga ctgaggaagg cctggagcag gggcagaggt     180

gacgacagtg cccctatatc cttaacccat actcctctga ggcaaacagg catgggaaaa     240

tggaagggtt gaggatggac cggagaattg gaacttcaga ataggtcaaa attccaaaac     300

catggacatt tttttttggg agaattgaga ttgtagacat tttttttttc ttaaatatga     360

tcaaggaaaa tagcttccag aatgtggtgg ttctgggcaa caaatgagat tgtggcgacg     420

tggagattaa aatatatgta tttgagctgg ggaatttgaa tattgtgagt ttcagatgtt     480

ggaaatttgg gattttgcag t                                               501

<210>  4
<211>  685
<212>  DNA
<213>  Artificial Sequence

<220>
<223>  EST 20693

<220>
<221>  misc_feature
<222>  (656)..(656)
<223>  "n" stands for unknown nucleic acid

<400>  4

cagtgagaaa aataaagttt atttcttgta cagaagtaat tccaattcag gttttaagtc 60

tgaaccttca aacttgggaa gtttttttcc aagtcatcag caagcgcctt atcaacttca 120

cgtaggattt taataaaatc ttccaccaca gggctataga aaggaagaag gttttgaaat 180

gcagaggaac acttctgttt gctcaactgg ccttccttta ctccctgagc caccgtctcc 240

tttttctatc tttagatact cttgctcagt ctgtgaggca tagatggcag acaacactga 300

aaaaacagag gctagcactg ttttctgttc ttgaagaatg atgggtggat catcagactg 360

gcagaattca tggcagacca ggtcaaaaag taaattccaa atgtcttttc cagtgtcgga 420

caatgtacaa ttgctgaatt cctcatccac tgtagtaagc agttgtaaaa tgtgctgtaa 480

acatcaagcc attctggatt ttcagaacgt acttgtttgg cagctcaagt atacagggca 540

caagccgaaa cactggctgc tctcagactc ttccggggtt ggtccagagg ctgagcagcc 600

cattctgacc catctaacat agttctcacc aatcaaggct gtcacactcc cacttnacag 660

cagcacatgt gactgctatg agcaa 685

<210> 5
<211> 495
<212> DNA
<213> Artificial Sequence

<220>
<223> EST 18658

<400> 5
actgttcgtt catttattca ttcaacaaac attatatgag cacccactat gtgccaggca 60

cagtgttagg tcccacacat acagagctga atcaggcata gtccttactt caggaatttc 120

acagtctagt agtagataca gacattttaa aaggtaatta taaaataaaa tggtaattat 180

ggtacaatac ggtaaatgaa ataatagaga tataccttgg gcattaagga accacaatag 240

agaggcatct acccaagcct agaatatagg acaggtttgc tggaagagct gggttctggg 300

ctgaataaac agcatgatat aagctgagaa ctaaaggtaa ttttgtccag agacagggaa 360

aggaggagtt aaggcatcag atgtaggcag gagggaggtc acagtggaat ttgaattaat 420

ctgtggcaca gcatttctgc ctggggtcaa gttggaggtc tgcagttata aattcttgga 480

tatacaagtt ttctg 495

<210> 6
<211> 99
<212> DNA
<213> Artificial Sequence

<220>
<223>  EST 1873

<400>  6
atattttatt caactttatt aaaaattaaa actacagaaa ccaaaccgaa tgcacaaata      60

agagagtggg gaaacagaca gggagcaatg gcagctgat                             99


<210>  7
<211>  484
<212>  DNA
<213>  Artificial Sequence

<220>
<223>  EST 20365

<400>  7
tttttcttc tcaaattttt aatggttctc cacggtatgc taggtcctta ttttctcatt      60

ggctgtaatt tgacaagttc tccaaaagca ctatcatgaa cttaattaga taatactttc     120

cagctgtact taaatcagaa ggatcgtagg cctgataagc aggtcaacct gaagctacca     180

ggcagctctg gaggctcccg tggccatagc taacagtgca caggagggcc cctggccagg     240

atgagccctc agaacctttt agagagagtg aaattcaggg tctactgggt gtccaagctg     300

cagagtggag cgtgagaggt cagctgccat gtgatagaaa gagctgacat aagagaaaag     360

acccccacac agtgaaaagc tggaagagaa acaaaactca caacctgaca gcagagttcc     420

tggttccaac tgttgagacc tgtctctacc tccaatggtc acagtatata gttacccagg     480

cctg                                                                  484

## Description

The invention relates to the field of diagnostics. More specifically the invention relates to determination of the presence and/or stage of a malignant cell.

Malignancies are a major cause of mortality world wide. Much research has been undertaken to gain insight into origin, development and characteristics of many different kind of malignancies. Based on this insight diagnostic and therapeutic methods have been developed. However, a malignancy at various stages requires different kind of treatment.

In view of the different characteristics of a malignancy at various stages, a reliable test for determining the presence, kind and/or stage of a malignancy is of utmost importance. Once the kind and stage of a malignancy is known, an optimal therapy can be chosen which sufficiently counteracts the malignancy while avoiding over-treatment in order to minimize harmful side-effects. However, the stage of a malignancy is not always reliably determined. For instance, morphologically grading of a malignancy by a pathologist does not always have a good reproducibility due to the subjective nature of morphological grading. The cellular morphology of malignant cells is highly variable in a significant number of cases and the malignant cell component is often heterogeneous within a biopsy.

Recently, a new method for diagnosing the presence and/or stage of malignancies has been developed. Expression profiles of a number of genes of malignancies are compared with reference expression profiles. The differences are used to evaluate the presence and/or stage of disease (WO 02/103320). This kind of diagnosis does not provide results for all kinds of malignancies. For instance, this kind of test does not provide results for determining the presence and/or stage of follicular lymphoma. According to the present invention, an expression profile of malignant cells and surrounding non-malignant cells is measured.

It is an object of the present invention to provide a method for detecting a malignant cell. It is a particular object to provide a method for staging a malignant cell.

The invention provides a method for detecting and/or staging a malignant functional cell comprising measuring an expression profile of said malignant cell and a non-malignant cell.

The invention provides the insight that the presence and/or stage of a malignant functional cell is determined by measuring an expression profile of said malignant cell and a non-malignant cell. Hence, although prior art methods focus on determining characteristics that are only attributable to a malignant cell, and which often distinguish said malignant cell from other non-malignant cells, the present invention provides the insight that an expression profile of a malignant functional cell and a non-malignant cell provides insight into the presence and, preferably, the stage of said malignant cell.

A malignant functional cell is defined herein as a malignant cell which is not dedifferentiated to such extent that all original functions except replication are lost. Said functional cell is still capable of performing at least one similar function ― in kind, not necessarily in amount ― as compared to the same kind of cell that is not malignant. Preferably, a malignant functional cell of the invention is still capable of interacting with adjacent tissue. In one embodiment adjacent, surrounding cells are capable of influencing at least one characteristic of said malignant functional cell. Preferably said adjacent, surrounding cells are capable of influencing proliferation of said malignant functional cell. In a further embodiment said malignant functional cell is capable of influencing at least one characteristic of said non-malignant cell. In one embodiment said malignant functional cell is capable of influencing at least one characteristic of an adjacent, surrounding non-malignant cell and an adjacent, surrounding cell is capable of influencing at least one characteristic of said malignant functional cell. In this embodiment an expression profile of said malignant cell and said non-malignant cell is preferably determined. In one embodiment an expression profile of said adjacent non-malignant cells is determined.

One typical example of a malignant functional cell of the invention is a follicular lymphoma cell. The natural course of waxing and waning disease in the early phases of follicular lymphoma indicates that the proliferation of follicular lymphoma cells is not fully autonomous and that the process is relatively dependent on exogenic factors, e.g. immunological drive. Clinical behavior in follicular lymphoma (FL) is partly determined by factors related to the malignant cells (genetic factors) and partly by non-malignant cells such as surrounding T-cells and immunological accessory cells. Follicular lymphoma cells are, at least in early stages, still capable of interacting with surrounding cells and they are still immunologically active, at least to some extent. Therefore, FL is a disease of immunologically functional cells. The growth properties are at least partially determined by immunological support.

Another typical example of a malignant functional cell of the present invention is a gastric marginal zone lymphoma of MALT-type (MALT-lymphoma). Gastric MALT-lymphoma derives from precursor populations in follicular *Helicobacter pylori*-associated gastritis, that through accumulating genetic changes ultimately develop into an autonomously proliferating, monoclonal B-cell lymphoma. In the early phases of the disease, the proliferation is still at least partly dependent on the presence of *Helicobacter pylori*-induced T-cell help as is supported by *in vitro* evidence as well as by *in vivo* evidence of clinical malignancy regression after eradication of *H.pylori*. Hence, at these stages a gastric MALT-lymphoma is not fully dedifferentiated and fully autonomous, but requires interaction with surrounding T cells for proliferation; such surrounding T cells are a good example of a non-malignant cell of the present invention.

Certain types of solid malignancies have retained at least part of their functional immunological properties and are therefore typical examples of a malignant functional cell of the invention. Renal cell carcinoma (Grawitz tumor) and melanoma often have functional antigen-presenting properties. Another preferred example of a malignant functional cell of the invention is a prostate cancer cell.

Staging of a malignant functional cell of the invention, also called grading of a malignant functional cell of the invention, means classification of a certain malignancy. The stage of a malignancy is determined. For instance, in case of follicular lymphoma, it is determined whether the malignancy is graded as 1, 2, 3a or 3b. With a method of the invention it has become possible to accurately stage a malignant functional cell. Especially when a follicular lymphoma has grade 2 or 3, a method of the invention is more accurate as compared to morphological grading. It has become possible to more reliably distinguish between FL that behave clinically indolent and those that behave aggressively. Hence, a method of the invention is also suitable when a biopsy with inconclusive morphological features is obtained, from which a pathologist cannot derive meaningful information for morphological grading. Once the stage is known, adapted therapy can be provided. Hence, a method of the invention allows for reliable diagnosis even in cases where morphological grading is ambiguous.

With a method of the invention an expression profile is measured. This means that it is determined whether at least one reporter gene is expressed by said malignant functional cell and/or said non-malignant cell. Preferably, expression of a plurality of reporter genes is examined. In one embodiment the invention provides a method of the invention comprising determining the presence and/or amount of an expression product of at least two reporter genes in a sample comprising said malignant functional cell and at least one non-malignant cell. Said malignant functional cell and said non-malignant cell are preferably sampled together. Preferably, a biopsy containing both said malignant cell and surrounding non-malignant cells is taken.

In one embodiment, a malignant functional cell is detected and/or staged by detecting a difference in expression of at least one reporter gene as compared to a reference and/or control. Said reference and/or control for instance comprises an expression profile of the same kind of functional cells that are not malignant and the same kind of non-malignant cells (herein called "healthy reference and/or control"). Alternatively, said reference and/or control comprises an expression profile of the same kind of malignant cells which have a specific, known, stage and in the same kind of non-malignant cells (herein called "stage reference and/or control"). An expression profile of a sample comprising said malignant cells and said non-malignant cells is compared to at least one reference and/or control. If an expression profile of a sample is significantly different from a healthy reference and/or control, it is indicative for the presence of malignant functional cells and/or the stage of said malignant functional cells. If an expression profile of a sample is essentially the same as a certain stage reference and/or control, it is indicative for the presence of malignant functional cells of the same stage. An expression profile of a sample is "essentially the same" if the amount of expression product of at least one gene of said sample involved in a certain pathway is comparable to the amount of expression product of at least one gene of said reference and/or control involved in the same kind of pathway. Preferably, the amount of expression product of at least one specific gene of said sample is comparable to the amount of expression product of the same kind of gene of said reference and/or control. Most preferably, the amount of expression product of a plurality of genes of said sample is comparable to the amount of expression product of the same kind of genes of said reference and/or control. In one embodiment, the amount of expression product present of a sample is quantified.

In a preferred embodiment an expression profile of at least one reporter gene which is involved with a proliferation/apoptosis pathway, a metabolism pathway, an immune competence pathway and/or a T cell infiltration pathway is measured, since genes involved with these pathways have a strong prognostic/diagnostic value in a method according to the invention.

In one embodiment said expression product comprises RNA, preferably mRNA. The amount of mRNA is particularly indicative for the extent of expression of a reporter gene. Many methods are known in the art for determining the presence of RNA. For instance, RNA is isolated from a sample, after which an amplification reaction such as Nasba or RT-PCR is performed. RNA is for instance detected and/or quantified using internal calibrators, Taqman probes and/or beacon probes. To achieve quantification, a dilution series of target sequence is for instance amplified and the time points at which amplified nucleic acid becomes measurable (the time to positivity, TTP) are plotted against the input amounts of nucleic acid. This way a calibration curve is created that is suitable for correlating TTP values of reactions with unknown amounts of input with input amount. Many alternative methods for detecting and/or quantifying RNA present in a sample are available in the art. Preferably, protocols for RNA isolation, amplification, labeling and hybridization as described at http://www.nki.nl/nkidep/pa/microarray are used. These protocols are incorporated in the examples.

In another embodiment said expression product comprises protein. An expression profile is for instance generated using western blot, (capture) ELISA, a (micro)array, etcetera.

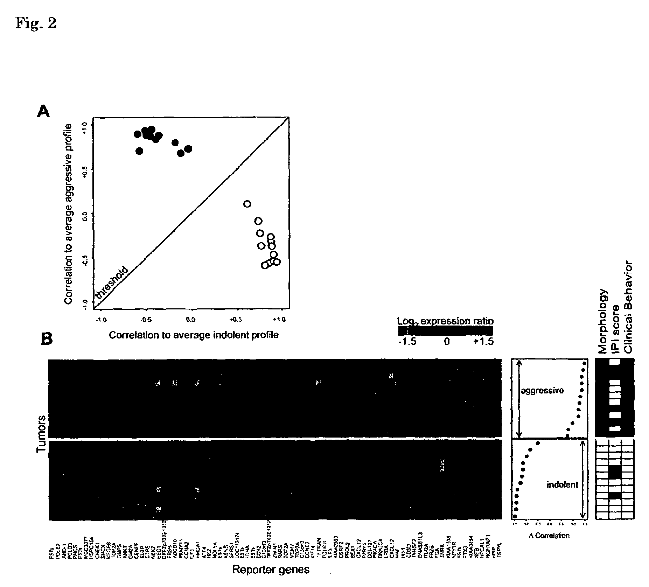

The invention provides as an exemplary embodiment a set of 81 reporter genes whose expression is correlated with the existence and stage of follicular lymphoma. This set of reporter gene is depicted in table 2. Each of said genes is differentially expressed in follicular lymphoma cells and surrounding non-malignant cells as compared to a healthy control of non-malignant B cells and surrounding non-malignant cells (non-malignant tonsillectomy specimens). The invention also provides a use of at least 5 of these markers for staging a follicular lymphoma. An expression profile of a sample comprising follicular lymphoma cells and/or non-malignant cells, preferably T cells, is determined. Preferably, said expression profile is compared with an expression profile of a control comprising non-malignant B cells and T cells. Most preferably, said expression profile is compared with an expression profile as depicted in figure 2B. An expression profile comparable to the upper part of figure 2B is indicative for an aggressive disease course while an expression profile comparable to the lower part of figure 2B is indicative for an indolent disease course. Similarity of expression profiles is for instance calculated using the Rosetta Error Model (Hughes TR, Marton MJ, Jones AR et al. Functional discovery via a compendium of expression profiles. Cell. 2000;102:109-126) and/or using the methods described on page 6 line 3 - page 7 line 19 of WO 02/103320. In one embodiment a method of the invention is provided, comprising determining in a sample comprising said non-malignant cell the presence and/or amount of expression product of at least 5 reporter genes listed in table 2. More preferably, the presence and/or amount of expression product of at least 10 reporter genes listed in table 2 is determined. Even more preferably, the presence and/or amount of expression product of at least 30 reporter genes listed in table 2 is determined. Even more preferably, the presence and/or amount of expression product of at least 50 reporter genes listed in table 2 is determined. Most preferably, the presence and/or amount of expression product of each of the reporter genes listed in table 2 is determined. The more expression products are determined, the more reliable is the assay.

In one embodiment the invention provides a method for staging a follicular lymphoma present in a sample, said sample further comprising non-malignant cells, comprising detecting a difference in the expression of a plurality of genes of said follicular lymphoma and said non-malignant cells relative to a control, said plurality of genes consisting of at least 5 genes listed in table 2. In preferred embodiments said plurality of genes consists of at least 10, 15, 20, 25, 30, 35, 40, 45, 50, 55, 60, 65, 70, 75, 80 and/or 81 of the genes listed in table 2. The greater the amount of genes, the more reliable is the assay. In one embodiment said expression profile comprises the presence and/or amount of RNA, preferably mRNA, of said genes listed in table 2.

In a preferred embodiment said non-malignant cell and said malignant cell are capable of interacting. According to the invention, an expression profile of a malignant functional cell and a non-malignant cell which are capable of interacting is particularly indicative for the presence and the stage of said malignant functional cell. Preferably, said non-malignant cell is present in the surroundings of said malignant cell, such that interaction is enabled. A preferred example of a non-malignant cell of the invention is a T cell infiltrated into, or in close vicinity to, a lymphoma.

A malignant functional cell of the invention is preferably capable of producing an antibody, enzyme and/or hormone. In a preferred embodiment said malignant functional cell comprises a B cell. Said B cell is preferably still capable of responding to activation by T helper cells and capable of producing antibody. The presence of a malignant B cell which is still functional, such as a malignant follicular lymphoma cell, results in an expression pattern of said B cell and a non-malignant cell such as a T helper cell which is indicative for the presence and stage of said malignant B cell. In one aspect a method of the invention is provided wherein said non-malignant cell comprises a T cell, preferably a T helper cell. Preferably, an expression profile of a follicular lymphoma cell and a surrounding T helper cell is determined. In order to efficiently stimulate B cells and CTL, a T helper cell is stimulated by an antigen presenting cell. Antigen presenting cells are also in the vicinity of a lymphoma. According to the present invention, an expression profile of a malignant functional cell and a surrounding non-malignant cell is indicative for the presence and stage of said malignant functional cell. In one embodiment, said expression profile comprises an expression profile of a lymphoma cell and an antigen presenting cell such as a dendritic cell.

Now that the invention has provided the insight that an expression profile of a malignant functional cell and a non-malignant cell is indicative for the presence and/or stage of said malignant cell, it has become possible to establish gene-expression profiles capable of detecting and/or staging a wide variety of malignant functional cells. Establishment of a gene-expression profile of 81 genes capable of detecting and staging follicular lymphoma is described in the examples. Likewise, an expression profile capable of detecting and/or staging another kind of malignant functional cells is established. Said expression profile is established by measuring an expression profile of said malignant functional cell and/or a non-malignant cell. Said non-malignant cell is possibly surrounding said malignant cell, such that interaction is possible. Preferably, an expression profile of a sample comprising said malignant functional cell and a non-malignant cell is determined and compared to a reference and/or control expression pattern. In one preferred embodiment said malignant functional cell comprises a mucosa-associated lymphoid tissue lymphoma cell. In another preferred embodiment said malignant functional cell comprises a renal cell carcinoma (such as a Grawitz tumor) and/or a prostate cancer cell. A set of reporter genes whose expression is correlated with the existence and stage of mucosa-associated lymphoid tissue lymphoma cell, renal cell carcinoma (such as a Grawitz tumor) and/or a prostate cancer cell is also herewith provided. Said sets are established using a comparable approach as outlined in the examples for follicular lymphoma. Contrary to prior art approaches, the use of malignant cells only for establishing an expression profile is avoided. Instead, a sample comprising non-malignant cells is used. Preferably, said non-malignant cells comprise cells capable of interacting with said malignant cells. More preferably, said non-malignant cells comprise cells surrounding said malignant cells, such as infiltrating T cells.

Reporter genes of the invention and their expression products are suitable drug targets. Expression of said reporter genes is influenced in order to treat malignant functional cells. For instance, expression of a reporter gene overexpressed in aggressive malignancy stages is at least in part reduced. This way aggressive malignant cells are at least in part counteracted, and/or conversion of a malignant functional cell from an indolent stage to an aggressive stage is counteracted. Preferably, a medicament is administered to an individual suffering from malignant functional cells which medicament is capable of at least in part counteracting expression of a reporter gene normally overexpressed in aggressive malignancy stages. In one embodiment a medicament is administered to an individual suffering from malignant functional cells which medicament is capable of at least in part enhancing expression of a reporter gene normally underexpressed in aggressive malignancy stages. In a further embodiment, a medicament is used that is capable of at least in part counteracting at least one function of an expression product of a reporter gene. For instance, a compound capable of specifically binding a protein encoded by said overexpressed reporter gene is suitable for counteracting said malignant functional cells. Binding of said compound to said protein at least in part inhibits a function of said protein. Said compound preferably comprises an antibody, single chain variable fragment and/or a Fab fragment. In one embodiment, said protein is associated to the surface of a malignant functional cell, since a drug directed to a surface-associated protein can more easily reach its target.

The invention furthermore provides an array comprising a compound capable of specifically binding an expression product of a reporter gene of the present invention. Said compound preferably comprises a nucleic acid sequence capable of specifically hybridizing to an RNA product of a reporter gene of the invention. Said nucleic acid sequence is preferably complementary to at least part of a reporter gene of the invention, said part being at least long enough for being specific for said particular reporter gene. Said part is preferably at least 30 base pairs long, more preferably at least 50 base pairs long, more preferably at least 70 base pairs long, more preferably at least 100 base pairs long, more preferably at least 150 base pairs long, most preferably at least 200 base pairs long. An increasing length contributes to the specificity of an assay performed with said array. In one embodiment an array comprising nucleic acid molecules capable of specifically binding to (an expression product of) at least 5 reporter genes listed in table 2 is provided. Preferably, said array comprises nucleic acid molecules capable of specifically binding to at least 10, more preferably 15, more preferably 20, more preferably 25, more preferably 30, more preferably 35, more preferably 40, more preferably 45, more preferably 50, more preferably 55, more preferably 60, more preferably 65, more preferably 70, more preferably 75, more preferably 80 (expression products of) reporter genes listed in table 2. Most preferably an array is provided comprising nucleic acid molecules capable of specifically binding to (expression products of) each of the reporter genes listed in table 2. The greater the amount of reporter genes, the more reliable an assay performed with said array is. Said expression products preferably comprise mRNA.

In one embodiment at least 50% of the probes on an array of the invention is capable of specifically hybridizing with (an expression product of) a reporter gene listed in table 2. In preferred embodiments, at least 60%, 65%, 70%, 75%, 80%, 85%, 90%, 93%, 95%, 97%, 98%, 99% or 100% of the probes on an array of the invention is capable of specifically hybridizing with a reporter gene listed in table 2. The greater the amount of probes capable of specifically hybridizing with (an expression product of) a reporter gene listed in table 2, the more reliable is the assay performed with said array.

In another preferred embodiment an array comprising a compound capable of specifically binding an expression product of a reporter gene of the present invention is provided, wherein said compound is capable of specifically binding at least part of a protein encoded by a reporter gene of the invention. Said compound preferably comprises an antibody, single chain variable fragment and/or a Fab fragment. In one embodiment an array comprising compounds capable of specifically binding proteinaceous molecules encoded by at least 5 reporter genes listed in table 2 is provided. Preferably, said array comprises compounds capable of specifically binding proteinaceous molecules encoded by at least 10, 15, 20, 25, 30, 35, 40, 45, 50, 55, 60, 65, 70, 75, 80 or 81 reporter genes listed in table 2. The greater the amount of reporter genes, the more reliable an assay performed with said array is.

In one embodiment at least 50% of the compounds on an array of the invention is capable of specifically binding proteinaceous molecules encoded by a reporter gene listed in table 2. In preferred embodiments, at least 60%, 65%, 70%, 75%, 80%, 85%, 90%, 93%, 95%, 97%, 98%, 99% or 100% of the compounds on an array of the invention is capable of specifically binding proteinaceous molecules encoded by a reporter gene listed in table 2. The greater the amount of compounds, the more reliable is an assay performed with said array.

The invention further provides an array for detecting and/or staging a malignant functional cell, comprising a positionally-addressable array of polynucleotide probes bound to a support, said polynucleotide probes comprising a sequence complementary and hybridizable to at least part of at least 5 reporter genes listed in table 2, said part being at least long enough for being specific for said particular reporter gene. Said part is preferably at least 30 base pairs long, more preferably at least 50 base pairs long, more preferably at least 70 base pairs long, more preferably at least 100 base pairs long, more preferably at least 150 base pairs long, most preferably at least 200 base pairs long. An increasing length contributes to the specificity of an assay performed with said array. An array of the invention preferably comprises a positionally-addressable array of polynucleotide probes bound to a support, said polynucleotide probes comprising a sequence complementary and hybridizable to at least part of at least 10, 15, 20, 25, 30, 35, 40, 45, 50, 55, 60, 65, 70, 75, 80, or 81 reporter genes listed in table 2. Preferably, at least 50% of the probes on the array is complementary to at least part of the reporter genes listed in table 2. Most preferably, at least 60%, 65%, 70%, 75%, 80%, 85%, 90%, 93%, 95%, 97%, 98%, 99% or 100% of the probes on the array is complementary to at least part of the reporter genes listed in table 2. In one embodiment an array is provided comprising at least one probe comprising at least part of a sequence as depicted in Table 2, said part being at least long enough for being specific for said sequence. Said part is preferably at least 30 base pairs long, more preferably at least 50 base pairs long, more preferably at least 70 base pairs long, more preferably at least 100 base pairs long, most preferably at least 150 base pairs long.

An array of the invention preferably comprises a microarray. An array of the invention is generated using known methods in the art such as for instance described on page 121 line 33 - page 122 line 19 of WO 02/103320 and/or according to Brazma A et al, Minimum information about a microarray experiment (MIAME)-toward standards for microarray data. Nat Genet. 2001;29:365-371.

An array of the present invention is particularly suitable for detecting and/or staging a malignant functional cell. In one embodiment a sample comprising said malignant cell together with non-malignant cells is brought into contact with an array of the invention. In one embodiment said sample is processed before it is brought into contact with an array of the invention. For instance, nucleic acid and/or protein is purified from said sample using known methods such as for instance the RNA purification protocol described in the examples, and/or cell lysis, centrifugation, chromatography and/or desalting (protein purification). After incubation of an array of the invention with (a purified part of) said sample, non-bound components are preferably washed away. Subsequently it is determined whether a binding pattern is obtained which is indicative for the presence and/or stage of said malignant functional cells. Binding of expression products of reporter genes is detected using known methods in the art, such as (radioactive or fluorescent) labeling of expression product. Since many methods for purifying and detecting expression products are available, a method of the present invention can be performed in many alternative ways.

In one aspect the invention therefore provides a method for detecting and/or staging a malignant functional cell comprising:
- providing an array of the present invention with a sample comprising expression products from said malignant functional cell and from a non-malignant cell; and
- determining whether an expression profile indicative for the presence and/or stage of said malignant functional cell has bound to said array.

Preferably said expression product comprises nucleic acid. In another preferred embodiment said expression product comprises protein. In one embodiment said malignant functional cell comprises a follicular lymphoma cell, a mucosa-associated lymphoid tissue lymphoma cell, a cell of a Grawitz tumour and/or a prostate cancer cell.

The invention has provided the insight that an expression profile of malignant functional cells and non-malignant cells is indicative for the presence and/or stage of said malignant functional cells. Said malignant cell and said non-malignant cell are preferably capable of interacting, at least at some stages of the disease. Without being bound to theory, it is believed that interaction between a malignant functional cell and another kind of cell influences both functioning of said malignant cell and functioning of said non-malignant cell. As a result, the expression profile of said malignant cell and said non-malignant cell changes. For instance, continuous stimulation by T helper cells enhances follicular lymphoma development. An expression profile of a sample comprising follicular lymphoma cells and T helper cells is indicative for the presence and/or stage of follicular lymphoma. Since a malignant functional cell of the invention is often still capable of interacting with other kind of cells, intervention between said interaction at least in part counteracts a disease involving a malignant functional cell of the invention.

The invention therefore provides a method for treating a disease involving a malignant functional cell, comprising at least in part inhibiting an interaction between said malignant functional cell and a non-malignant cell. Preferably, signaling of a T cell is at least in part counteracted. Said T cell preferably comprises a T helper cell. Signaling of a T cell is defined as influencing at least one characteristic of a malignant functional cell of the invention by said T cell. Said characteristic for instance comprises replication and/or the production of antibodies. Signaling of a T cell for instance comprises secretion of cytokines.

Said interaction between said malignant functional cell and said non-malignant cell is for instance at least in part inhibited by a compound capable of binding said malignant cell, said non-malignant cell, and/or a compound involved with said interaction such as for instance a cytokine. Said compound for instance comprises an antibody, single chain variable fragment and/or a Fab fragment. Administration of said compound at least in part inhibits proliferation of a malignant cell and, hence, is used as a medicament. The invention thus provides a compound capable of at least in part inhibiting signaling of a T cell for use as a medicament.

Said compound is suitable for treating a disease involving a malignant functional cell. A use of a compound capable of at least in part inhibiting signaling of a T cell for the preparation of a medicament for treating a disease involving a malignant functional cell is therefore also herewith provided.

In one embodiment said disease comprises follicular lymphoma, mucosa-associated lymphoid tissue lymphoma, a Grawitz tumour and/or prostate cancer. Said compound is preferably capable of at least in part counteracting an interaction between a malignant B cell and a T helper cell. Non limiting examples of suitable compounds are CXCR-4 inhibitors and fludarabin.

### Detailed description

One kind of malignancy-related disease which involves staging problems is follicular lymphoma. Follicular lymphomas (FL) form a well defined clinicopathological entity and are the second most frequent lymphoma in adults. In the Netherlands, approximately 2000 new cases of non-Hodgkin's lymphoma are seen yearly, of which 20% are FL.

The t(14;18) translocation lies at the basis of oncogenesis in follicular lymphoma. Follicular lymphoma is a disease characterized by a long clinical course for most patients and marked by frequent relapses that vary in clinical aggressiveness over time. The median overall survival varies from 6-12 years, but the clinical course of this disease is highly variable. Some patients have waxing and waning disease for several years without any treatment. Generally, the malignancy is chemo- and radiosensitive with remission rates of 70-80%. However, the disease inevitably relapses and curative treatment is at present not possible.

Transformation to diffuse large B-cell lymphoma (DLBCL) is a common event and occurs in approximately 80-85% of the patients. Primary treatment may vary from watchful waiting to high dose chemotherapy with stem cell transplantation with the aim to reach long lasting disease-free survival and in a small subset of patients possibly cure. At subsequent relapse, treatment is again stratified for clinical aggressiveness. Transformation and development of unresponsiveness to chemotherapy in the course of the disease form the main causes of death in patients with FL and in these situations more aggressive and possibly experimental therapy is justified. In individual patients and at each relapse the diagnosis of the phase of the disease (indolent or aggressive) is important for the choice of optimal therapy. Timing of the most optimal treatment is of particular importance for the benefit of patients, since both over-treatment and under-treatment in relation to the clinical aggressiveness of the disease affects event-free survival and overall survival. Unnecessary or premature use of anthracyclin is harmful with respect to quality of life and morbidity, and the use is limited by an absolute maximum tolerable dose. Conversely, withholding aggressive chemotherapy (in clinically aggressive disease) will result in insufficient therapy response with consequences for disease-free and overall survival, especially in the first line.

Thus far, morphological sub-classification has been used as a major guide in the choice of therapy in FL. In the World Health Organization Classification (WHO), FL is graded as 1, 2, 3a and 3b according to the number of large transformed cells (centroblasts) per high-power field (Nathwani BN, Harris NL, Weisenburger D et al. In: Jaffe,E.S.; Harris,N.L.; Stein,H.; Vardiman,J.W.,eds. Pathology and genetics of tumours of haematopoietic and lymphoid tissues. Lyon: IARC Press; 2001). Even though these criteria appear objective, grading has a notoriously poor reproducibility among community pathologists, as well as experienced hematopathologists (agreement 61-73%). This is due to the subjective nature of morphological grading and the inherent inadequacy of the criteria set for "transformed cells". The cellular morphology of transformed cells may be highly variable and the component of transformed cells may be heterogeneous within the biopsy. Therefore, classical morphological grading results in too many inconclusive cases (10 to 30% of all cases) and is not optimally suitable as a guideline in the choice of therapy in daily practice. An alternative method with objective and reproducible parameters to judge clinical aggressiveness at any decision point for treatment in patients with FL is therefore desired. Clinical prognostic indices, such as the International Prognostic Index (IPI) (A predictive model for aggressive non-Hodgkin's lymphoma. The International Non-Hodgkin's Lymphoma Prognostic Factors Project. N Engl J Med. 1993;329:987-994; Hermans J et al, Blood. 1995;86:1460-1463) and variations that are more tailored to FL (FLIPI) (Federico M et al, Intergruppo Italiano Linfomi. Blood. 2000;95:783-789; Solal-Celigny P and Roy P. Ann Oncol. 2002;13:Abstract 54) are in principle suitable markers of clinical course. However, the discriminative value for the majority of patients is limited since most patients will fall in the low and low-intermediate risk groups due to the limitations and resolution of the system. Moreover, these indices are only formally validated for predictive value at diagnosis. Hence, other means of stratification are desired.

The invention is further illustrated by the following examples. The examples do not limit the scope of the invention in any way.

### Brief description of the drawings

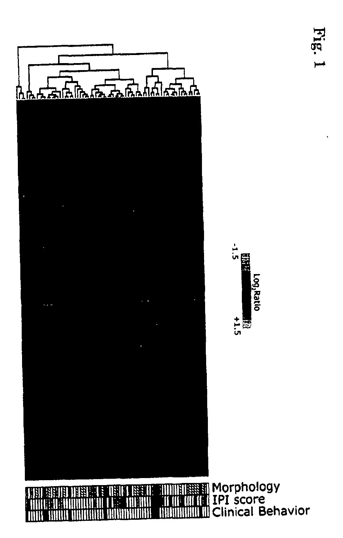

**Figure 1. Unsupervised clustering of 72 FL samples, grades 1, 2 and 3, shows a relative biological homogeneity of FL**. There is a separation in three main groups with no enrichment for morphological grades or clinical behavior in either of the groups. Each row represents a malignancy and each column a single gene. Gene-expression is depicted according to the color-bar. Red indicates a high level of mRNA-expression relative to the reference and green indicates a low level of expression. Selected morphological and clinical data are depicted in the right panel. For clinical features: white indicates indolent; black indicates aggressive disease behavior (see Table 1 for criteria). For morphological grading: white indicates grade 1, dashed grade 2, black grade 3. For IPI: white denotes scores 0,1 or 2, black 3, 4 or 5. Gray denotes insufficient data.
**Figure 2. Supervised classification of 12 paired lymphoma samples (training series).**
   A. Correlation plot of the FL-stratification profile. Correlation of the expression profile of each malignancy sample with the average expression profile of all "indolent" samples is depicted on the vertical axis, and the correlation of the expression profile of each malignancy with the average expression profile of all "aggressive" malignancy samples is shown on the horizontal axis. Malignancies classified above the threshold are classified as molecular high-grade; malignancies below the threshold are classified as molecular low-grade.
   B. Expression data matrix of 81 marker genes from malignancies of the indolent as well as the aggressive phase of 12 follicular lymphoma patients. Each row represents a malignancy and each column a gene. Genes are ordered on the basis of their SNR. Maliganncies are rank-ordered according to the difference in correlation with the average "high-grade profile" and the correlation with the "low-grade profile" (middle panel). The solid yellow line is the classifier with optimal accuracy; above the yellow line patients have an aggressive disease course, below the yellow line, patients have an indolent disease course at the disease episode at the time of biopsy. Selected clinical data is shown in the right panel: Morphological data, white indicates FL grade 1 or 2; black indicates FL grade 3B or DLBCL; IPI scores and clinical behavior at time of biopsy as in Fig 1.

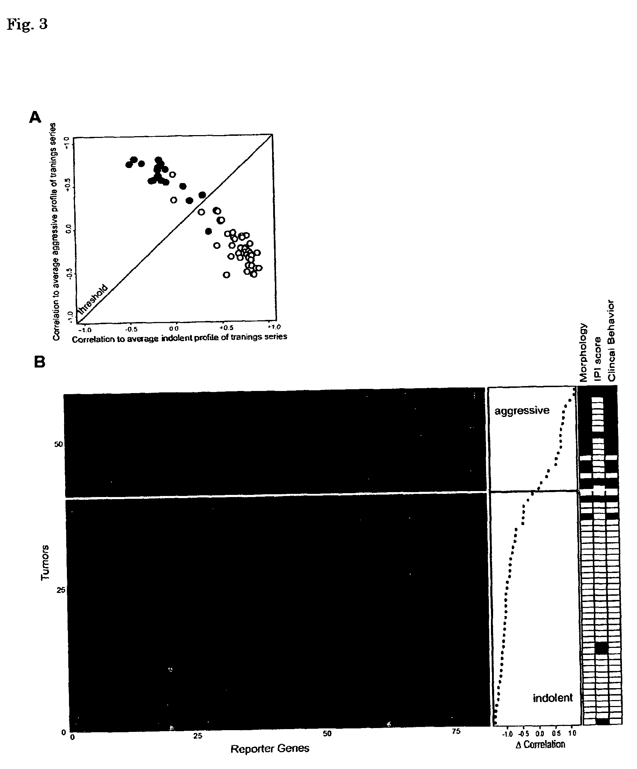

**Figure 3. Correlation and pattern of expression of genes used to determine the clinical characteristics of 58 lymphoma samples (independent validation series).**
   A. Correlation plot of the FL-stratification profile. Correlation of the expression profile of each malignancy sample with the average expression profile of all "low-grade" samples of the training series is depicted on the vertical-axis, and the correlation of the expression profile of each malignancy with the average expression profile of all "high-grade" malignancy samples of the training series is shown on the horizontal-axis. Malignancies classified above the threshold are classified as molecular high-grade; malignancies below the threshold are classified as molecular low-grade.
   B. Expression data matrix of 81 marker genes from malignancies of the indolent as well as the aggressive phase of follicular lymphoma patients. Each row represents the FL-stratification profile for one malignancy. Each column represents the relative expression of one gene. The genes in the horizontal direction are arrayed in the same order as in Figure 2B. Malignancies are rank ordered according to the difference in correlation with the average "aggressive" profile and the correlation with the "low-grade profile" of the training set (middle panel). The yellow line is the threshold as determined in Figure 2 (see Figure 2 for color scheme). In the panel on the right morphological and clinical features are shown of the samples similar to features shown in Figure 2B. Of the 58 samples, 4 were misclassified (93% accuracy).

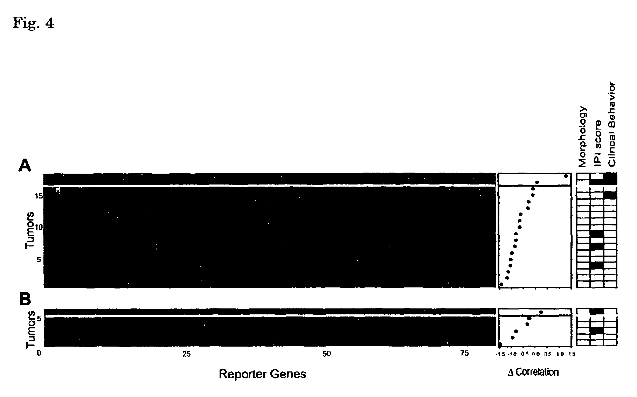

**Figure 4. Pattern of gene expression of genes used to determine the clinical characteristics of 24 Follicular Lymphoma samples (test series)**. Panel A shows the expression data matrix of 18 malignancy samples with an unclear morphology across the 81 gene profile. The genes in the horizontal direction are arrayed in the same order as in Figure 2B. Malignancies are rank ordered according to the difference in correlation to the molecular high-grade and low-grade profile as in Figure 2 (middle panel). The yellow line is the threshold as determined in Figure 2B (see Fig 2 for color scheme). Morphological data, IPI scores and Clinical behavior are shown on the right. White, gray and black as indicated in Figure 1B. Of the 18 samples, the classifier predicted the actual clinical behavior correctly in 17 patients (accuracy 94%). Panel B shows expression data of six malignancy samples with unequivocal morphological and clinical indolent disease, but who presented with full-blown aggressive disease within 10 months. One sample was classified as molecular high-grade.

### Examples

### Example 1

### Methods

### Design of the study

The aim of this study was to search for a gene-expression profile that assesses indolent versus aggressive clinical behavior both at diagnosis and at relapse for the disease episode at the moment of biopsy to replace current inadequate morphological and clinical methods. Since adequate upfront criteria and gold standards for clinical behavior are not available, the complete course of the disease was evaluated retrospectively for all patients and each disease episode was defined as either indolent (non-aggressive) or aggressive. To qualify as a clinically non-aggressive or indolent disease episode, the following criteria had to be met: 1) absence of B symptoms, elevated LDH, rapid generalized disease progression in the preceding 3 months, and 2) if treated, a good malignancy response (partial or complete remission) after at least one of two non-anthracyclin-containing, non-high dose chemotherapy regimens (e.g. chlorambucil, CVP, or fludarabin). An aggressive disease episode was defined as development of B-symptoms and LDH and/or rapid generalized disease progression within the preceding 3 months, and/or progressive disease during successive treatment with two or more non-anthracyclin, non-high dose chemotherapy regimens (Table 1). In case of ambiguous clinical situations, the patient was not included in this study. Since FL is a genetically very homogeneous disease and the variation in expression patterns is within small limits, a supervised approach was chosen. A gene-expression profile was developed in a set of paired samples and validated in a separate series of patients. Most importantly, the profile was tested in a third series of cases with ambiguous morphological features.

### Patient selection

Malignancy samples from patients with primary nodal follicular lymphoma treated between 1984 and 2002 were selected from the fresh-frozen-tissue banks of the Netherlands Cancer Institute, Central Laboratories Friesland, University Medical Center Nijmegen, Leiden University Medical Center and Groningen University Medical Center according to the following criteria: availability of a representative frozen sample and paraffin-embedded tissue, proven diagnosis of FL and availability of complete clinical data at presentation and during follow-up. The study was approved by the medical-ethics committee of the Netherlands Cancer Institute.
106 samples from 80 patients were included in this study. Of these patients, the full medical history from diagnosis to last follow-up or death was evaluated retrospectively including data on IPI parameters (A predictive model for aggressive non-Hodgkin's lymphoma. The International Non-Hodgkin's Lymphoma Prognostic Factors Project. N Engl J Med. 1993;329:987-994), treatment and treatment results. All relevant biopsy material was reviewed, including full immunohistochemical workup (CD20, CD3, CD5, bcl-2, bcl-6, CD10, CD21) by three hematopathologists (DdJ, RK, JvK), classified and graded according to the WHO classification. A selection of clinical and histological data is summarized in Supplementary Table 1. IPI parameters are grouped in a low/low-intermediate risk group for IPI score 0, 1 or 2, and a high-intermediate/high-risk group for IPI score 3, 4 or 5. For each patient indolent and aggressive disease episodes were assigned in retrospect according to the criteria described in the design of the study. It should be noted that for the retrospective clinical classification histological grading was not included. All patients for whom due to lack of data the clinical episode of the available biopsy sample could not be reliably assigned to the indolent or aggressive phase of the disease, were excluded.
Treatment varied and was administered according to local protocols at the time of diagnosis, including cyclophosphomide/vincristine/prednisone (CVP), fludarabin, chlorambucil, chlorambucil/prednisone, and cyclophosphamide/doxorubicin/vineristine/prednisone (CHOP-like) with or without radiotherapy. Different "indolent-type" treatment protocols have been shown to be equally effective in FL and do not result in different disease-free and overall survival (Horning SJ. Follicular lymphoma: have we made any progress? Ann Oncol. 2000;11 Suppl 1:23-27; Horning SJ. Natural history of and therapy for the indolent non-Hodgkin's lymphomas. Semin Oncol. 1993;20:75-88). Since the aim of the study is to find a profile related to clinical behavior at the time of biopsy and not a survival-related or treatment-response related profile, variability in treatment will not influence our study results.

The patient samples were divided in three different groups.
**1) Training series:** Patients of whom paired samples corresponding to the concordant clinical/morphological indolent and to concordant clinical/morphological aggressive episodes were available; i.e., combined indolent disease course and FL grade 1 or 2 versus aggressive episode and FL grade 3B or DLBCL (Nathwani BN et al. In: Jaffe,E.S.; Harris,N.L.; Stein,H.; Vardiman,J.W.,eds. Pathology and genetics of tumours of haematopoietic and lymphoid tissues. Lyon: IARC Press; 2001). Twenty-four paired samples of both phases in 12 patients were used to build the classifier profile and to minimize patient specific variation. Age at diagnosis of these selected patients ranged from 24 to 74 years (median 49.5 years, median follow up 80 months, range 29-304 months). Transformation to aggressive disease occurred with a median interval of 64 months (range 22-288 months).
**2) Validation series**: 58 independent samples (54 patients) of clinical/morphological indolent phases or clinical/morphological aggressive phases. Age at diagnosis ranged from 27 to 78 years (median 53 years). The median duration of follow-up was 71 months (range 4 to 206 months). The aggressive phase samples consisted of 18 patient samples, median interval to transformation 36.5 months, range 3-139 months. The samples of the indolent group consisted of 18 patient samples (median follow-up 91 months, range 24-68 months) as well as 22 samples from the indolent phases previous to transformation (median interval to transformation 47 months, range 21-168 months).
**3) Validation (series) for difficult cases:** Eighteen patient samples were selected that showed ambiguous morphological features according to the review panel (DdJ, RK, HvK) and were scored as inconclusive in the past: borderline between FL grade 2 and grade 3A or a histology characterized by the dominant presence of small centroblast-like cells that fell outside the consensus criteria for classical "large transformed cells" ((Nathwani BN et al. In: Jaffe,E.S.; Harris,N.L.; Stein,H.; Vardiman,J.W.,eds. Pathology and genetics of tumours of haematopoietic and lymphoid tissues. Lyon: IARC Press; 2001).
   In addition, 6 patients were selected who presented with a FL grade 1 and clinically indolent behavior in the first biopsy, but showed histological transformation to DLBCL and progression to clinically high-grade and aggressive disease within 10 months (median time to progression 6.5 months, range 3 to 10 months).

### RNA Isolation, amplification and labeling and hybridization:

Detailed protocols for RNA isolation, amplification, labeling and hybridization can be found at http://www.nki.nl/nkidep/pa/microarray and are presented above. All samples were co-hybridized with a standard reference of pooled and amplified RNA isolated from tonsillectomy specimens of 5 patients who underwent routine tonsillectomy for chronic ear/nose/throat-infections. This tissue reference was chosen to provide a lymphoid reference containing all genes that are potentially expressed in the malignancy tissues at a significant level and biologically closely related to the malignancy samples to be able to identify small changes in expression levels between the malignancy groups.

### Microarray slides:

Microarray slides were prepared at the central microarray facility (CMF) at the Netherlands Cancer Institute. Sequence verified cDNA clones (InVitrogen, Huntsville, AL) were spotted onto poly-L-lysine-coated glass slides using the Microgrid II arrayer (Apogent, Cambridge, UK) with a complexity of 19,200 spots per slide. A complete list of genes and controls included on the slides is available on the CMF web site (http://microarrays.nki.nl), as well as details on the process of preparing the DNA for spotting and preparation of the slides.

### Normalization:

Fluorescent intensities were normalized and corrected for a variety of biases that affect the intensity measurements (e.g., color bias and print tip bias) according to Yang *et al*. (Yang YH, Dudoit S, Luu P et al. Normalization for cDNA microarray data: a robust composite method addressing single and multiple slide systematic variation. Nucleic Acids Res. 2002;30:e15). Weighted averages, and confidence levels were computed according to the Rosetta Error Model (Hughes TR, Marton MJ, Jones AR et al. Functional discovery via a compendium of expression profiles. Cell. 2000;102:109-126).

### Unsupervised clustering:

Gene clustering and malignancy clustering were performed independently using agglomerative hierarchical clustering in the software program genesis (Sturn A, Quackenbush J, Trajanoski Z. Genesis: cluster analysis of microarray data. Bioinformatics. 2002;18:207-208). For gene clustering, complete linkage similarity metrics among genes were calculated on the basis of expression ratio measurement across all malignancies. Similarly, for malignancy clustering complete linkage clustering was calculated based on expression ratio measurements across all significant genes.

### Supervised classification:

To reliably discriminate between the indolent and aggressive malignancies, the following supervised classification method was used to build the FL-stratification classifier:
1) From the 19,200 genes on the microarray, genes that were significantly different from the reference in at least two malignancies, were selected (significance was based on the P-values (p<0.01) computed with the Rosetta Error Model (Hughes TR, Marton MJ, Jones AR et al. Functional discovery via a compendium of expression profiles. Cell. 2000;102:109-126)).
2) Calculation of the paired signal-to-noise-ratio (SNR) for each of the selected genes, and ranking of the genes (top ranked genes being genes that are best suited to separate indolent from aggressive samples).
3) Determination of top ranked genes that separate the two classes best when employed in a nearest prototype classifier (van 't Veer LJ, Dai H, van de Vijver MJ et al. Gene expression profiling predicts clinical outcome of breast cancer. Nature. 2002;415:530-536). Steps 2 and 3 are performed in a cross-validation procedure, where at each cross-validation iteration a matched pair is left out and employed to test the performance of the classifier trained on the remaining pairs.

The optimal number of genes that could separate molecular low-grade from molecular high-grade disease was determined in a leave-pair-out-cross-validation method. More specifically, in 12 repetitive steps, each paired sample was left out once, and for the remaining 22 samples, genes were ranked on the SNR. Then starting with the four most informative genes the classifier was trained on the 22 samples and employed to predict the outcome of the left-out pair. The classification of the left-out pair was predicted based on the largest Pearson correlation of the expression profile of each of the left-out samples with the mean expression levels of the remaining samples from the "indolent" or the "aggressive" patient samples for the selected reporter genes. Subsequently, the whole procedure was repeated by adding one gene at a time. Note that in order to avoid selection bias (Simon R et al, J Natl Cancer Inst. 2003;95:14-18; Ambroise C et al, Proc Natl Acad Sci U S A. 2002;99:6562-6566), i.e. underestimation of the error rate, samples that were left out were not involved in *any* of the reporter selection steps.
The performance was measured as the average of the false positive and negative rates of the left-out samples. No further increase in performance was observed when the number of included reporter genes exceeded 81. Since the ranking in every cross-validation iteration produces a slightly different set of marker genes, the final set of 81 classifier genes in the FL-stratification classifier was determined by taking the 81 genes that occurred most frequently in each of the 12 steps.

**Additional microarray information.** The description of this study followed the MIAMI guidelines issued by the Microarray Gene Expression Data group (Brazma A, Hingamp P, Quackenbush J et al. Minimum information about a microarray experiment (MIAME)-toward standards for microarray data. Nat Genet. 2001;29:365-371).

### Results:

To provide an accurate and clinically relevant classification of FL, 106 specimens from 80 patients (supplementary Table 1) were analyzed for gene-expression profiles using 19K cDNA microarrays.
As a first step, unsupervised two dimensional hierarchical clustering was performed on 72 FL grade 1, 2, 3a and 3b samples to group genes on the basis of similarity across all malignancies and to group the malignancy samples according to similarities in gene expression (Fig.1). This shows a relative homogeneity of FL as a single disease entity and shows a division in three main groups with no enrichment for morphological grade or clinical behavior. It should be noted, that multiples samples of single patients do not dominantly cluster together and both IPI-score and treatment are no component in the clustering. Unsupervised clustering is by far insufficient for clinical use, therefore, a supervised classification approach was chosen.

### Development of a molecular profile for low-grade and high-grade disease in FL on the basis of paired samples

To build a classifier a group of 24-paired samples from 12 patients was used for whom samples of both the clinically and morphologically indolent phase as well as the clinically and morphologically aggressive phase of the disease were available. Using the Rosetta Error Model (Hughes TR et al. Cell. 2000;102:109-126), a total of 4760 mRNAs were found to be differentially expressed compared to the reference RNA (P-value<0.01 in 2 or more samples). From these, a set of 81 genes emerged from the cross-validation procedure with the optimal classification performance of 100%.
The correlation coefficients for each of the malignancy samples with the average expression of these 81 genes in either "low-grade" or "high grade" samples in the cross-validation were calculated and are shown in Fig. 2a. Malignancies positioned above the threshold were classified as aggressive; malignancies below the threshold were classified as low-grade. The line of equal correlation was chosen as threshold since misclassification in both directions (false positives and false negatives) would result in equally adverse treatment consequences for the patient.
The expression pattern of the 81 genes in the 24 learning samples is shown in the color plot of Fig 2b, where the malignancies are ranked according to the difference in correlation with the average "high-grade" profile and the correlation with the "low-grade profile" (middle panel).
Expression-data were associated with data on clinical and histological parameters (histological grading, IPI score and clinical classification (Fig. 2b, right panel) and correlated perfectly with histological grading and clinical behavior. This is not unexpected since the tumors were selected for unambiguous morphological features concordant with clinical behavior. Importantly, IPI score was not found to be a very strong discriminative factor for clinical behavior.
The FL-stratification profile (Fig. 2*b*) contains genes significantly up-regulated in the aggressive phase of the disease that are involved in cell cycle control (e.g. CCNE2, CCNA2, CDK2, CHEK1, MCM7) and DNA-synthesis (e.g. TOP2A, POLD3A, HMGA1, POLE2, GMPS, CTPS) as well as genes reflecting increased metabolism (FRSB, RARS, HK2, LDHA). Genes involved in signal transduction reflecting activation of several signaling pathways are differentially expressed (e.g. FRZB, HCFCR1, PIK4CA, MAPK1). Genes derived from the reactive infiltrate of T-cells and macrophages (CD3D, CXCL12, TM4SF2) were up-regulated in the indolent phase of the disease as expected from immunohistochemical data from corresponding paraffin-embedded material of all samples, showing 40-70% T-cell infiltration in FL and 20-50% T-cell infiltration in DLBCL.

### Validation of the FL-stratification profile in an independent series of transformed FL

To validate the classifier, an additional independent set of 58 FL samples was investigated: 40 morphological/clinical indolent samples and 18 samples from the morphologically/clinically aggressive phase of the disease. Like the training series, cases were selected for concordant histological grade and clinical behavior. The gene-expression ratios of the 81 genes for these malignancies and their correlation coefficients of the malignancy samples with the average expression of these 81 genes with either "indolent" or "aggressive" samples of the training series are shown in Figure 3. The 18 samples with values above the threshold were assigned to the high-grade category; the 40 samples below the threshold were assigned to the low-grade category. When compared to morphological and clinical data (Fig. 3b), this resulted in 4 incorrect out of 58 classifications: two false negatives, i.e. morphologically/clinically aggressive samples classified as molecular low-grade and 2 false positives, i.e. morphologically/clinically indolent classified as molecular high-grade (93% accuracy).

### Performance of the FL-stratification profile in histologically difficult cases.

To further confirm the performance of the FL-stratification classifier, 18 diagnostically difficult cases with ambiguous morphological features were selected that precluded a meaningful decision (based on grade) by the pathologist; clinical behavior was determined in retrospect as in the training and validation series. The actual clinical course as predicted by the FL-stratification classifier revealed 2 samples classified as high-grade and 16 samples as low-grade. One of these samples was incorrectly classified; this patient was clinically assigned as having aggressive disease whereas the FL-stratification classifier predicted low-grade disease (Fig. 4, Panel A). Of these 18 patients IPI scores at the time of biopsy indicated low/low-intermediate risk in 10 patients (Fig. 4*a*, right panel white bars) and high/high-intermediate risk in 4 patients (black bars); in 4 patients no scores could be assigned due to lack of data (gray bars).
To evaluate the notion that the 81-gene profile reflects the actual clinical behavior at the time of biopsy rather than predicts future transformation, an additional set of six patients was selected who had morphologically evident indolent, grade 1 disease in their biopsy samples and had indolent disease course at the time of the biopsy, but who presented with full-blown morphologically and clinically aggressive disease within a period of 10 months. The classifier marked 5 of 6 cases as low-grade disease in support of this hypothesis.

We have developed a FL-stratification gene expression profile to assess the actual clinical behavior in follicular lymphoma to replace current insufficient morphological and clinical methods. The classifier of 81 genes was developed in a paired-samples series of 12 FL patients who later transformed to aggressive disease to discriminate at 100% accuracy. In an independent validation series of 40 patients in a morphological/clinical indolent disease phase and 18 patients with a morphological/clinical aggressive disease phase, the molecular profile showed an accuracy of 93%. Only samples with unequivocal morphological features of indolent FL (FL grade 1 or 2) and aggressive disease (FL grade 3B or DLBCL morphology) were included, to select for an expression profile that is optimized for genes that are involved in clinically relevant transformation to the aggressive phase of the disease. The profile is therefore most relevant in the stratification of FL. This profile applies to FL and transformed FL including DLBCL, but excludes de novo DLBCL. It should be noted that de novo DLBCL represents a different disease.

The selection of the morphological spectrum of samples used in the training and the validation set (FL grade 1 and 2, and FL grade 3B and DLBCL) has a good reproducibility among hematopathologists and accounts for the significant correlation of morphological stratification and clinical course in large series. However, in practice, the largest problem is in "borderline" cases that show heterogeneity with respect to the number of large transformed cells or that contain blast-like cells with a variant morphology. In these patients, who represent approximately 10-30% of all FL biopsies, the pathologist cannot provide meaningful information to base treatment strategies on. In view of the frequent relapsing nature of the disease, histological grading problems are encountered in the far majority of the patients. We show that the genes selected in a profile that is based on the extremes of the spectrum of FL, is also of predictive relevance in the morphologically "gray zone". Our molecular profile proves it's value mostly in this group of FL, as was supported by the findings in a test series containing this type of ambiguous cases, in which our FL-stratification profile adequately assessed the clinical behavior at the disease episode at the moment of biopsy in 17/18 patients. Notably, of the 16 patients that were classified as molecular low-grade, 5 of these patients were over-treated with aggressive therapy if currently used methods to guide therapy are compared to the stratification profile (data not shown). IPI scores in these patients showed mainly low and low-intermediate risk scores that would not add significantly as a guide in the choice of therapy. Our gene-expression-based profile outperforms IPI classification in all cases. Especially in difficult cases, the profile outperforms histological grading and significantly adds to the meaningful stratification of FL patients, and prevents patients from unnecessary aggressive treatment.

Functional annotation for the 81 genes in the FL-stratification classifier and in the full set of genes that are differentially expressed in indolent and aggressive disease provides more insight into the biological mechanisms underlying transformation in FL. Genes involved in cell cycle control, DNA synthesis and metabolism are significantly up-regulated in the aggressive phase of the disease as expected. The higher density of the T-cell infiltrate in low-grade FL as compared to high-grade disease is reflected by several T-cell related genes (CD3, CD2, CD69). However, genes related to T-cell and macrophage activation including several chemokine receptors (CCR1, CCL3, CCL5, CCL8, AKAP12, ILF3, GEM) are significantly upregulated upon transformation, indicating an important biological role. Notably, specific antagonists to several of the above mentioned chemokine receptors are available and offer attractive therapeutic interventions as do other individual gene products that are differentially expressed in the aggressive phase for which specific antagonistic and agonistic compounds have been developed (e.g. to the negative regulatory LPA-receptor Edg-2).

Our data show that on the basis of our FL-stratification profile, we are now capable of more reliably stratifying FL for low-grade and high-grade disease than by morphological grading and clinical prognostic parameters. The FL-stratification profile provides an important improvement and is an essential aid to guide the choice of therapy in FL patients to provide optimal disease control, optimal failure-free survival and quality-of-life.

RNA Isolation from frozen tissue 1

RNA isolation from frozen tissue

Before you start isolating RNA, make sure your workplace is RNAse free. Use
RNAseZap to clean your workplace. Wear gloves and use RNAse free
eppendorf tubes and pipette tips.

1. Cut 30 sections of 30 μm (Cryo-microtome @ –20°C) and transfer to tube
containing 1 ml RNAzol, shake vigorously and put on ice.
2. Homogenize samples using a Polytron Homogenizer
• 5-10 sec homogenize
• Shake tube
• 5-10 sec homogenize
3. Before use and between samples clean the Polytron by rinsing with
• RNAse free H2O (2x)
• RNAseZap (1x)
• RNAse free H2O (1x)
4. Spin samples 10 minutes at 14000 rpm and 4°C
5. Transfer supernatant (~1 ml) to fresh tube
6. Add 100 μl chloroform and mix thoroughly for 15 seconds
7. Keep samples on ice for 5-10 minutes
8. Spin samples 15 minutes at 12000rpm and 4°C
9. Transfer aqueous layer to a fresh tube
10. Add 600 μl isopropanol and mix by vortexing shortly
11. Precipitate samples at -70°C for 1 hour, or O/N at 4°C
12. Carefully pour off supernatant and add 1 ml 70% Ethanol to wash the pellet.
(the pellet should come loose from the tube)
13. Spin 10 minutes at 12000 rpm and 4°C
14. Pour off ethanol and spin briefly (5 seconds), use a pipette tip to remove the
remaining ethanol
15. Resuspend the RNA carefully in 200 μl RNAse free H2O
16. Measure RNA concentration (use 10 μl RNA and 490μl H20)
17. Run 1 μg on gel to check RNA quality
18. Add 20 μl 3M NaAc, pH 5.6 and 600 μl 100% EtOH and store at -70°C

Molecular Pathology - Netherlands Cancer Institute Amsterdam 09/03
www.nki.nl/pathology/microarrays a.glas@nki.nl

mRNA amplification by in vitro transcription 1

mRNA amplification by in vitro transcription
(V5.4 June 2003)

adapted from Chris Berry-NCI,
R. Kerkhoven- NKI,
Wang et al, Nat.Biotechnol 2000,
Van Gelder et al PNAS 1990,
Eberwine J, PNAS 1992.

Annuska Glas- NKI/AvL Amsterdam

A.glas@nki.nl
+31(20) 5122757

Molecular Pathology - Netherlands Cancer Institute Amsterdam v5.4
www.nki.nl/pathology/microarrays a.glas@nki.nl

mRNA amplification by in vitro transcription 2

1st strand synthesis:

• Total RNA 4 µg
Trehalose 6.7 µl (1.8 M)
Linamp oligo 1 µl (1µg/µl)
H2O (RNAse free) till 10µ l
10 µl

• 10' @ 65°C
On ice

• Prepare 1st strand mix:
1st strand buffer 4 µl (5x)
DTT 2 µl (0.1 M)
RNAsin 1 µl
dNTPs 1 µl ( 10 mM ea)
Linear Acrylamide 1 µl (0.1 µg/µl)
Superscript II Rev Tr 1 µl
10 µl

Add 1st mix to RNA/Linamp oligo
Total vol. 20 µl

• Thermocycler: (program T7-rna-1st)
37°C 5'
45°C 5'
then 10 cycles of 60°C 2'
55°C 2'
4°C -o-

Keep reaction on ice till you add 2nd strand mix

NOTE: Trehalose is added to enlarge the length of cDNA by increasing the reaction temperature
and therefore decreasing secondary structures in the RNA.

Linamp oligo: 5'-GGC CAG TGA ATT GTA ATA CGA CTC ACT ATA GGG AGG CGG TTT TTT TTT
TTT TTT TTT TTT TTT-3' (PAGE purified)

Trehalose | 10 g | Fluka | Cat# 90208
Ribonuclease inhibitor (RNAsin) | 1000 units | Gibco | Cat# 15518-012
Linear acrylamide | 5 x 1 ml | Ambion | Cat# 9520
SuperscriptII rev transcriptase | 10000 units | Gibco | Cat# 18064-014
dNTPs | 25 µM ea | Amersham | Cat# 27-2035-01
0.1M DTT and 1st strand buffer are included with Superscript II enzyme kit

Molecular Pathology - Netherlands Cancer Institute Amsterdam v5.4
www.nki.nl/pathology/microarrays a.glas@nki.nl

mRNA amplification by in vitro transcription 3

2nd strand synthesis

• Prepare 2nd strand mix:
H2O (RNAse free) | 91 µl |
2nd strand buffer | 30 µl | ( 5 x)
dNTPs | 3 µl | (10 mM ea)
E. Coli DNA ligase | 1 µl | (10 u/µl)
E. Coli DNA polym I | 4 µl | (10 u/µl)
RNAse H | 1 µl | ( 2 u/µl)
130 µl

• Add 2nd strand mix to 20 µl 1st strand product (total vol. 150 µl)

• 2 hrs @ 16°C

• 10'@ 65°C

• Use Qiagen PCR micro clean up columns to purify ds cDNA product.
Add 5 vol buffer PB to product
Transfer to Qiagen column
Spin 1 minute 14k rpm, discard flow through
Add 750 µl buffer PE
Spin 1 minute 14k rpm, discard flow through
Add 750 µl buffer PE or 80% EtOH
Spin 1 minute 14k rpm, discard flow through
Spin additional minute to dry membrane
Apply collection tube to column
Add 16 µl Elution buffer (buffer EB, or Tris pH 8)
Spin 1 minute max speed (14k rpm)

The cDNA can be stored @ -20°C at this point

10 x second strand buffer | 0.5 ml | Gibco | Cat# 10812-014
E. Coli DNA ligase | 100 units | Gibco | Cat# 18052-019
E. Coli DNA Polymerase | 1000 units | Gibco | Cat# 18010-025
RNAse H | 250 units | Promega | Cat# M4285
QIAquick PCR Purification Kit | 50 | Qiagen | Cat# 28104

Molecular Pathology - Netherlands Cancer Institute Amsterdam v5.4
www.nki.nl/pathology/microarrays a.glas@nki.nl

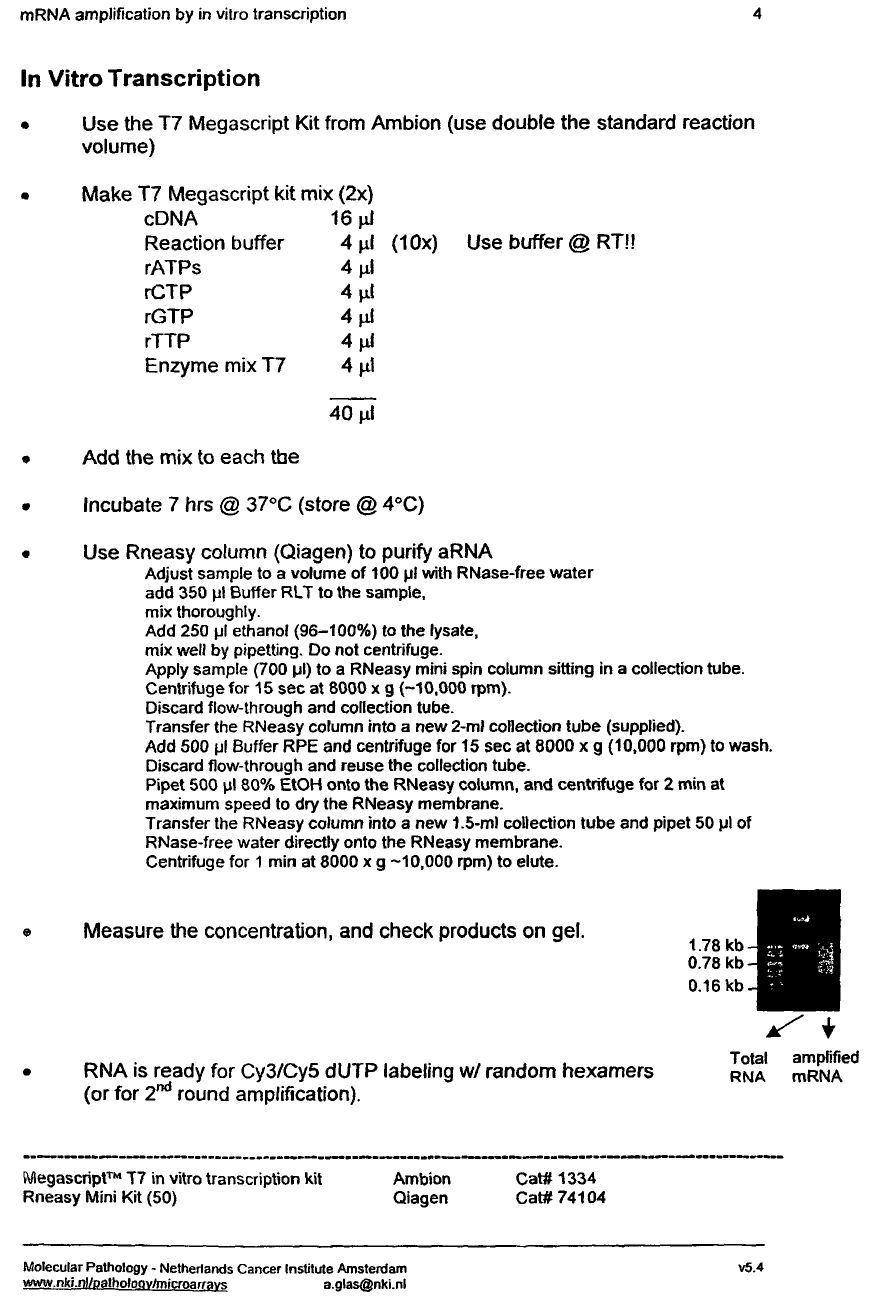
mRNA amplification by in vitro transcription 4

In Vitro Transcription

• Use the T7 Megascript Kit from Ambion (use double the standard reaction
volume)

• Make T7 Megascript kit mix (2x)
cDNA | 16 µl
Reaction buffer | 4 µl | (10x) | Use buffer @ RT!!
rATPs | 4 µl
rCTP | 4 µl
rGTP | 4 µl
rTTP | 4 µl
Enzyme mix T7 | 4 µl

40 µl

• Add the mix to each tbe

• Incubate 7 hrs @ 37°C (store @ 4°C)

• Use Rneasy column (Qiagen) to purify aRNA
Adjust sample to a volume of 100 µl with RNase-free water
add 350 µl Buffer RLT to the sample,
mix thoroughly.
Add 250 µl ethanol (96–100%) to the lysate,
mix well by pipetting. Do not centrifuge.
Apply sample (700 µl) to a RNeasy mini spin column sitting in a collection tube.
Centrifuge for 15 sec at 8000 x g (~10,000 rpm).
Discard flow-through and collection tube.
Transfer the RNeasy column into a new 2-ml collection tube (supplied).
Add 500 µl Buffer RPE and centrifuge for 15 sec at 8000 x g (10,000 rpm) to wash.
Discard flow-through and reuse the collection tube.
Pipet 500 µl 80% EtOH onto the RNeasy column, and centrifuge for 2 min at
maximum speed to dry the RNeasy membrane.
Transfer the RNeasy column into a new 1.5-ml collection tube and pipet 50 µl of
RNase-free water directly onto the RNeasy membrane.
Centrifuge for 1 min at 8000 x g ~10,000 rpm) to elute.

• Measure the concentration, and check products on gel.

1.78 kb
0.78 kb
0.16 kb
Total
RNA
amplified
mRNA

• RNA is ready for Cy3/Cy5 dUTP labeling w/ random hexamers
(or for 2nd round amplification).

Megascript™ T7 in vitro transcription kit | Ambion | Cat# 1334
Rneasy Mini Kit (50) | Qiagen | Cat# 74104

Molecular Pathology - Netherlands Cancer Institute Amsterdam v5.4
www.nki.nl/pathology/microarrays a.glas@nki.nl

Fluorescent Probe labeling and Hybridization.doc
1

Fluorescent Probe (aRNA) labeling and
Hybridization to cDNA MicroArrays.
(V5.4 jun 2003)

adapted from R. Kerkhoven- NKI and Chris Berry-NCI

Annuska Glas- NKI/AvL Amsterdam

a.glas@nki.nl
+31(20) 5122757

Molecular Pathology-Netherlands Cancer Institute Amsterdam
www.nki.nl/pathology/microarrays a.glas@nki.nl
v5.4

Fluorescent Probe labeling and Hybridization.doc 2

Fluorescent Probe (aRNA) labeling for microarray hybridization

• mix aRNA (amplified antisense RNA) 2 µg
random hexamer pd(N)6 3 µg
H2O till 21 µl

• Incubate 10 min @ 70°C

• Add:
5x Superscript 1st strand buffer 8 µl
0.1 M DTT 4 µl
0.5 mM dA/C/G TP, 0.2 mM dTTP 2 µl
RNAsin 1 µl
Cy3 or Cy5 dUTP 2 µl
Superscript II 2 µl

• Incubate 1hr @ 42°C, add 1 µl Superscript II

• Incubate 1hr @ 42°C then place on ice

• Purify the probes using a Qiagen mini-elute PCR clean up colum
1. Add 5 volumes buffer PB to labeled probe
2. Transfer to Qiagen column
3. Spin 1 minute 14k rpm, discard flow through
4. Add 750 µl buffer PE (alternatively use 80% EtOH)
5. Spin 1 minute 14k rpm, discard flow through
6. Repeat steps 4 and 5 one more time
7. Spin additional minute to dry membrane
8. Apply collection tube to column
9. Add 10 µl Elution buffer (buffer EB, or Tris pH 8)
10. Spin 1 minute max speed (14k rpm)

Alternatively purify probes using a Qiagen PCR clean up column
Step 1- 8 as above
9. Add 50 µl Elution buffer (buffer EB, or Tris pH 8)
10. Spin 1 minute max speed (14k rpm)
Then perform an EtOH precipitation to reduce volume
Add 3 µg COT-1 DNA as carrier
Add 5 µl SodiumAcetate (3 M, pH 5.6)
Add 150 µl EtOH (100%)
Mix by vortexing
Store in -20°C for 15 minutes
Spin 20' MAX speed
Wash pellet with 150µl EtOH (70%)
Resuspend in 5 µl RNAse free H2O

Molecular Pathology-Netherlands Cancer Institute Amsterdam v5.4
www.nki.nl/pathology/microarrays a.glas@nki.nl

Fluorescent Probe labeling and Hybridization.doc
3

Random Hexamers | 20 µg | Pomega | Cat# C1181
SuperscriptII rev transcriptase | 10000 units | Gibco | Cat# 18064-014
Cy3 dUTP | 25 nmol | Amersham | Cat# PA55022
Cy5 dUTP | 25 nmol | Amersham | Cat# PA53022
RNAsin | 1000 units | Gibco | Cat#15518-012
dNTPs | 25 µM ea | Amersham | Cat# 27-2035-01
MinElute PCR Purification Kit | 50 | Qiagen | Cat# 28004
Qiaquick PCR purification Kit | 50 | Qiagen | Cat# 28104

Molecular Pathology-Netherlands Cancer Institute Amsterdam
v5.4
www.nki.nl/pathology/microarrays
a.glas@nki.nl

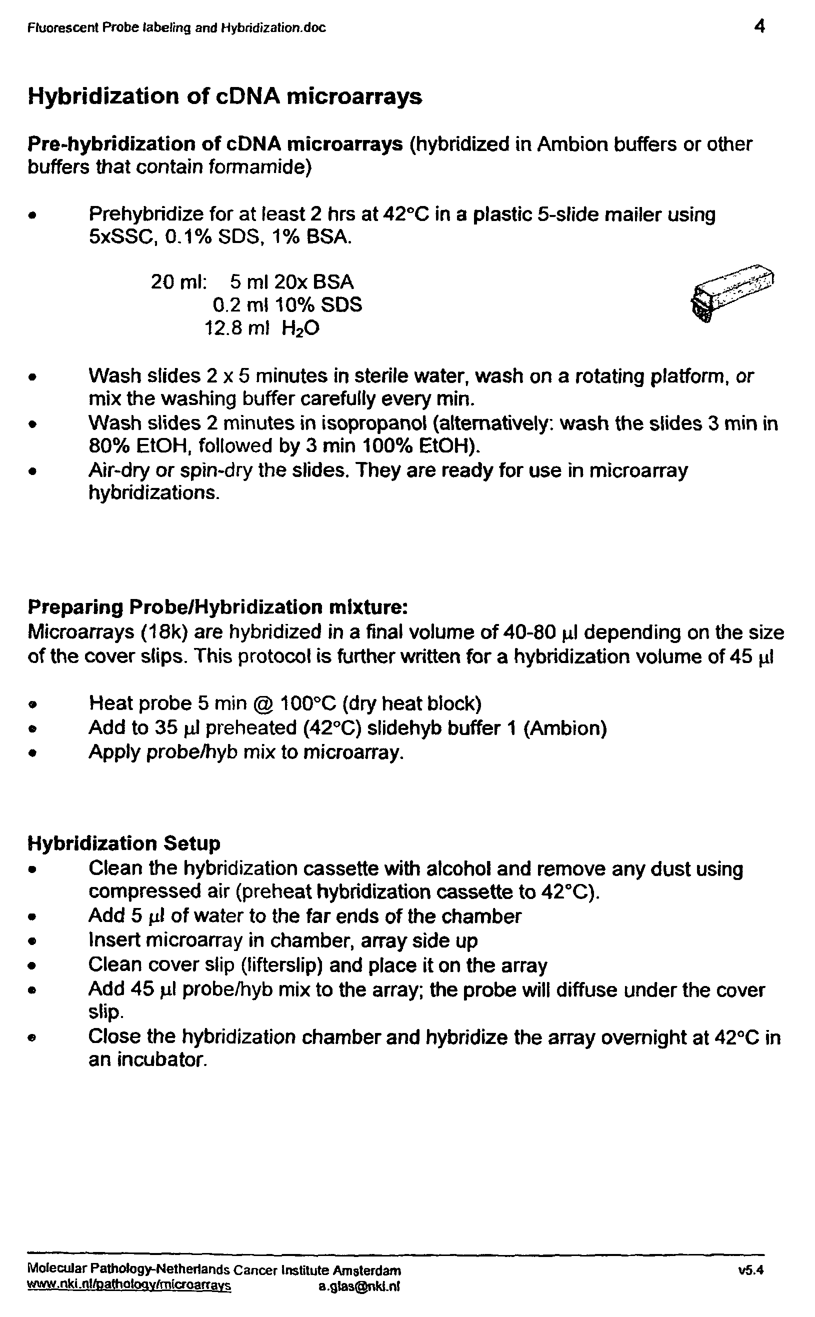
Fluorescent Probe labeling and Hybridization.doc 4

Hybridization of cDNA microarrays

Pre-hybridization of cDNA microarrays (hybridized in Ambion buffers or other
buffers that contain formamide)

• Prehybridize for at least 2 hrs at 42°C in a plastic 5-slide mailer using
5xSSC, 0.1% SDS, 1% BSA.

20 ml: 5 ml 20x BSA
0.2 ml 10% SDS
12.8 ml H2O


• Wash slides 2 x 5 minutes in sterile water, wash on a rotating platform, or
mix the washing buffer carefully every min.
• Wash slides 2 minutes in isopropanol (alternatively: wash the slides 3 min in
80% EtOH, followed by 3 min 100% EtOH).
• Air-dry or spin-dry the slides. They are ready for use in microarray
hybridizations.

Preparing Probe/Hybridization mixture:
Microarrays (18k) are hybridized in a final volume of 40-80 µl depending on the size
of the cover slips. This protocol is further written for a hybridization volume of 45 µl

• Heat probe 5 min @ 100°C (dry heat block)
• Add to 35 µl preheated (42°C) slidehyb buffer 1 (Ambion)
• Apply probe/hyb mix to microarray.

Hybridization Setup
• Clean the hybridization cassette with alcohol and remove any dust using
compressed air (preheat hybridization cassette to 42°C).
• Add 5 µl of water to the far ends of the chamber
• Insert microarray in chamber, array side up
• Clean cover slip (lifterslip) and place it on the array
• Add 45 µl probe/hyb mix to the array; the probe will diffuse under the cover
slip.
• Close the hybridization chamber and hybridize the array overnight at 42°C in
an incubator.

Molecular Pathology-Netherlands Cancer Institute Amsterdam v5.4
www.nki.nl/pathology/microarrays a.glas@nki.nl

Fluorescent Probe labeling and Hybridization.doc 5

Hybridization washes

• Following the hybridization reaction remove the slide from the cassette.
• Quickly transfer the slide to a 50 ml tube containing preheated (42°C) wash
solution 2x SSC, 0.1%SDS. The cover slide will slide down the array slowly.
• Transfer the array to a glass slide rack with an open bottom and immerse in
a staining dish containing 1xSSC, wash 2 times 5 min at 42°C
Perform the washes on a rotating platform, or mix the washing buffer
carefully every min.
• Wash 1 x 5 min in 0.2xSSC (buffer preheated to 42°C).
• Wash app. 30 sec in 0.05xSSC (RT)
• Spin-dry the array 3 min @ 600 rpm (or air dry)
• Scan the array immediately (if possible, otherwise store slide dark and dust
free).

NOTES:
Prior to pre-hybridization, filter all solutions. Salt particles in the SSC might cause
bad spots on the array and 10% BSA is an ideal growth medium for bacteria.

Make sure you wash the slides thoroughly in water after pre-hybridization. Any
BSA that is still on the slide causes a lot of Cy3 background in the hybridization,
which cannot be washed away.

When the hybridization solution does not cover the slide completely, you may push
the cover slip gently using a pipette tip to make sure the solution will spread evenly
over the array.

Slidehyb buffer 1 | 5x2 ml | Ambion | Cat# 8861
Hybridization casettes | ea | Ambion | Cat# 10040
Lifterslips | | Erie Scientific | Cat#25x60I-2-4789

Molecular Pathology-Netherlands Cancer Institute Amsterdam v5.4
www.nki.nl/pathology/microarrays a.glas@nki.nl

**Table 1: Criteria for retrospective assessment of the actual clinical behavior of at any stage of the disease in follicular lymphoma patients.**

| **Indolent clinical behavior** | **Aggressive clinical behavior** |
|---|---|
| Slowly progressive or waxing and waning disease during watchful waiting during minimally 3 months | Rapid progression/enlargement of a tumor mass at one or more localizations within 3 months |
| **OR** | **AND/OR** |
| Upon treatment: good tumor response (CR*/PR⁺)³⁶) after treatment with CVP≠ or chlorambucil or chlorambucil/prednisone or fludarabin in first line or second line. | Development of B-symptoms and LDH elevation after previously normal levels in the preceding 3 months |
| **AND** | **AND/OR** |
| Normal levels of LDH ^{§} and no B-symptoms" | Progressive disease during successive treatment with two or more of the following treatment protocols CVP, chlorambucil, chlorambueil/prednisone and fludarabin. |

| | |
|---|---|
| *CR: Complete remission; +PR: partial remission; | |
| ‡CVP: cyclophosphomide, vincristine, prednisone; §LDH: lactate dehydrogenase; | |
| "B-symptoms: more than 10% weight loss within 6 months, night sweats, fever more than 38°C. | |

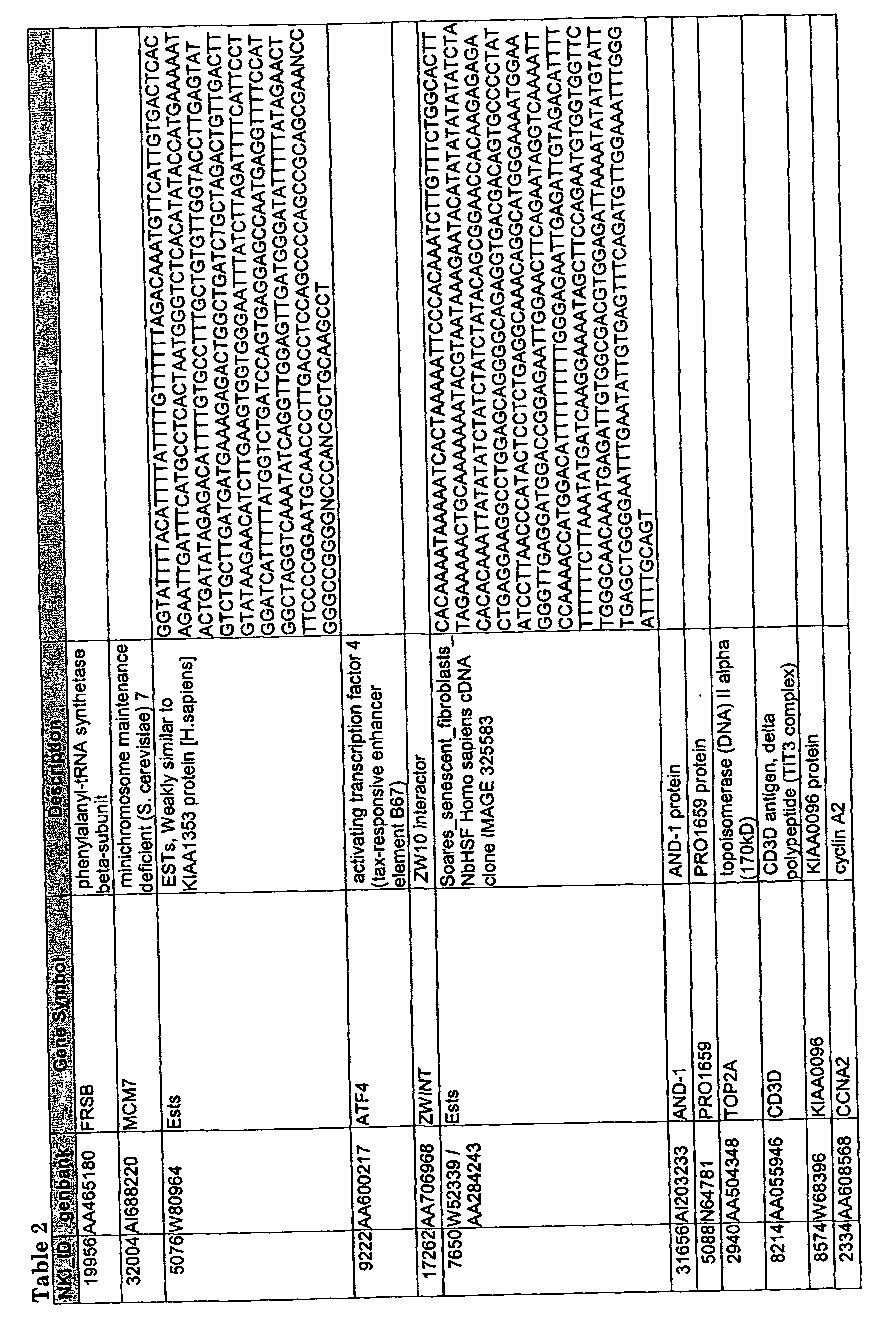

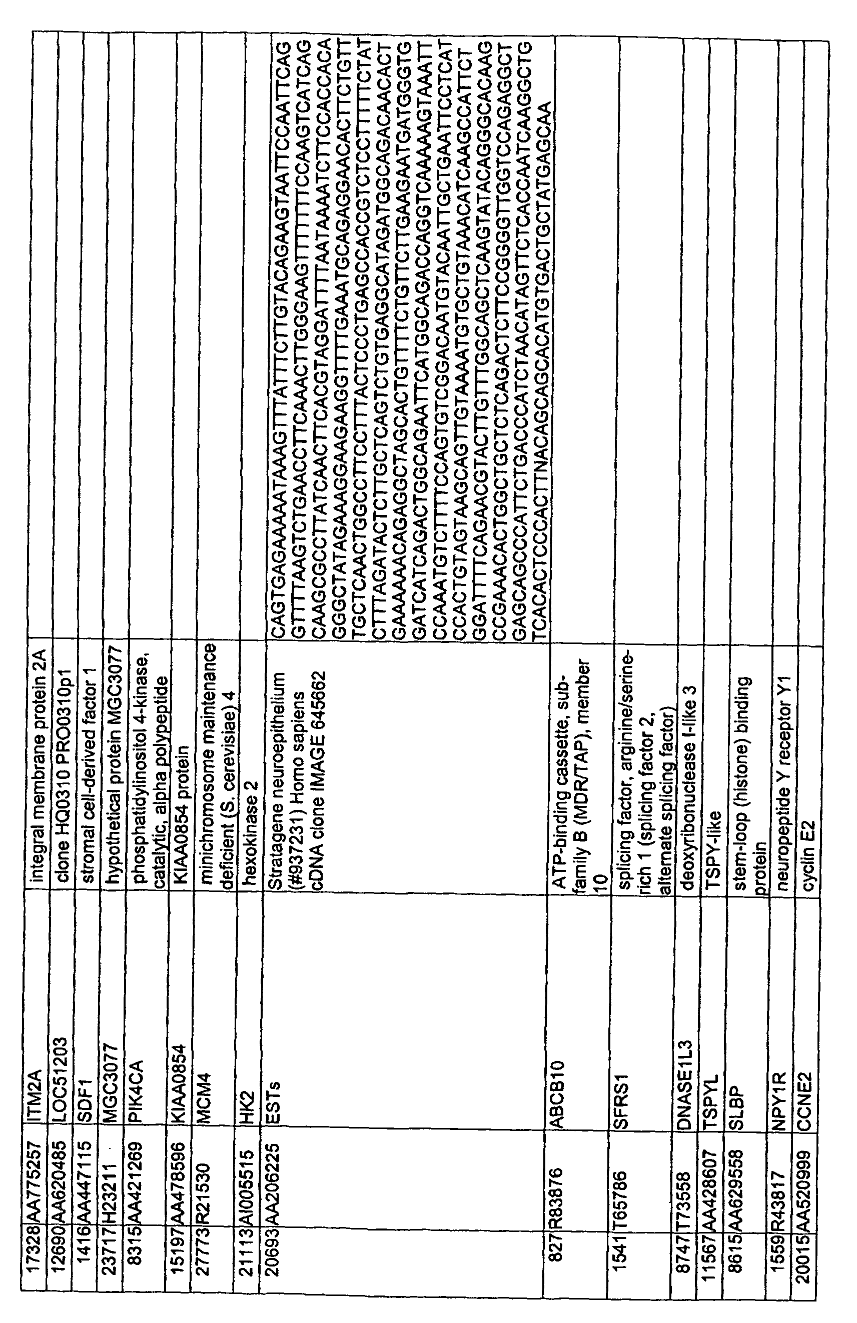

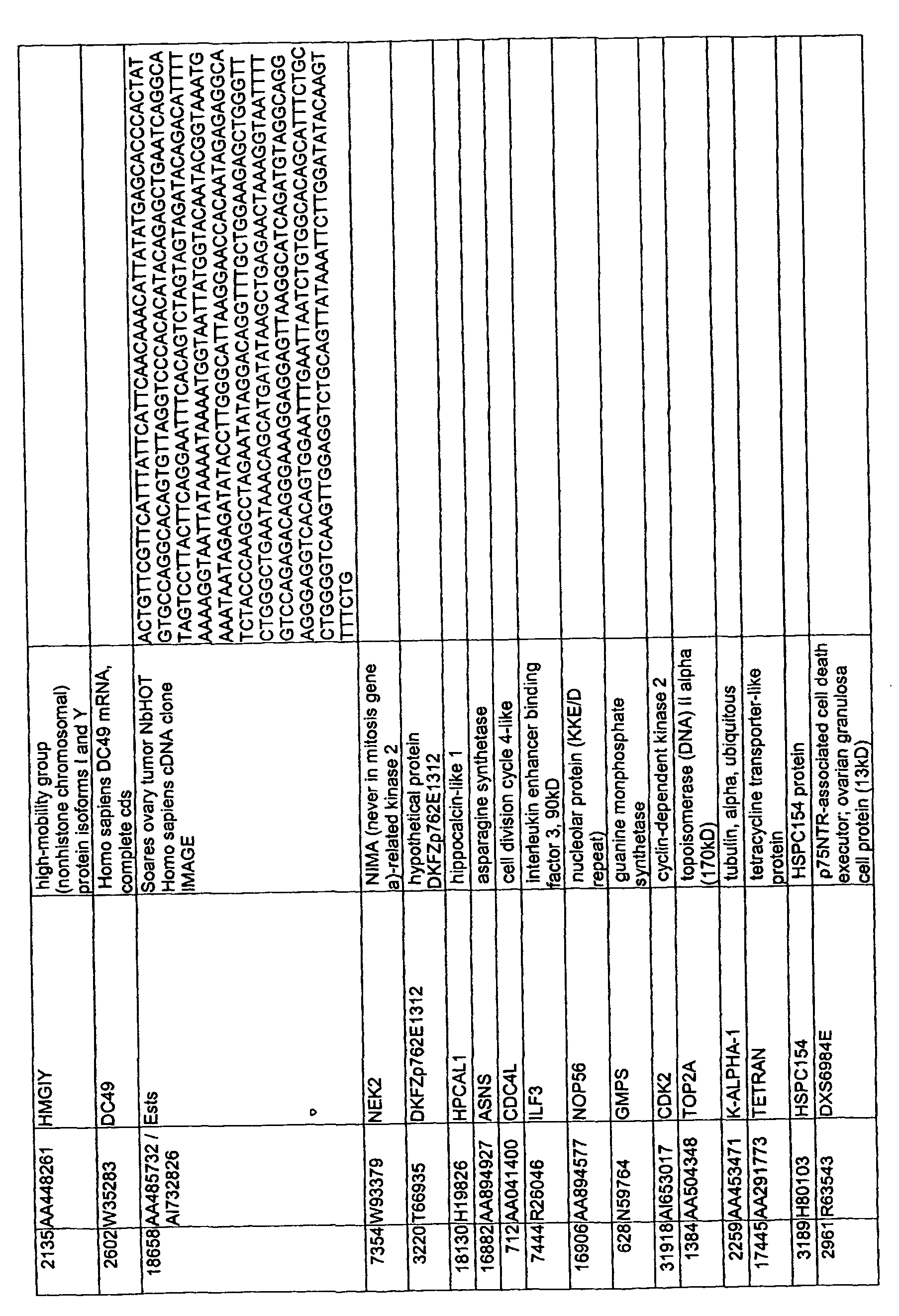

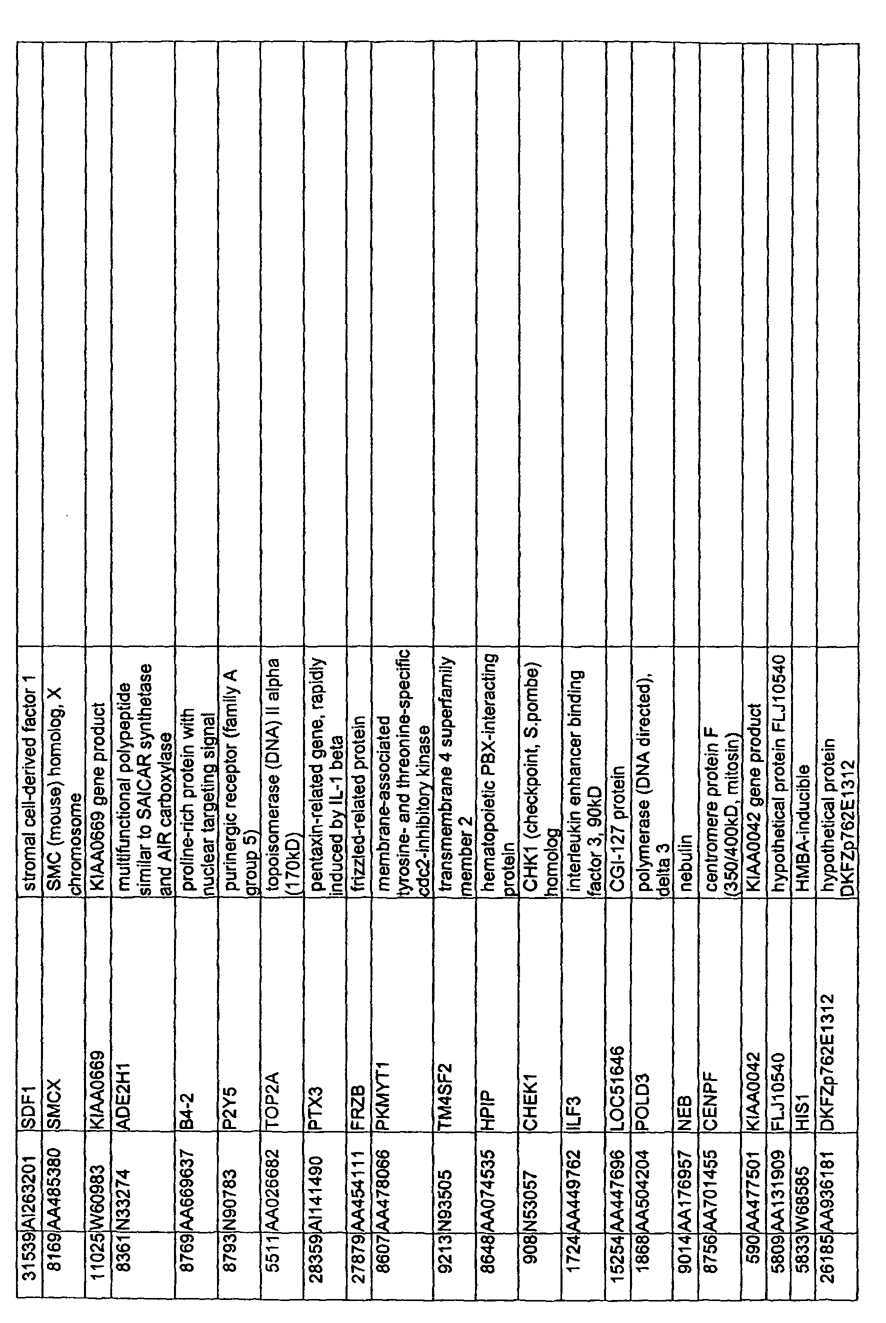

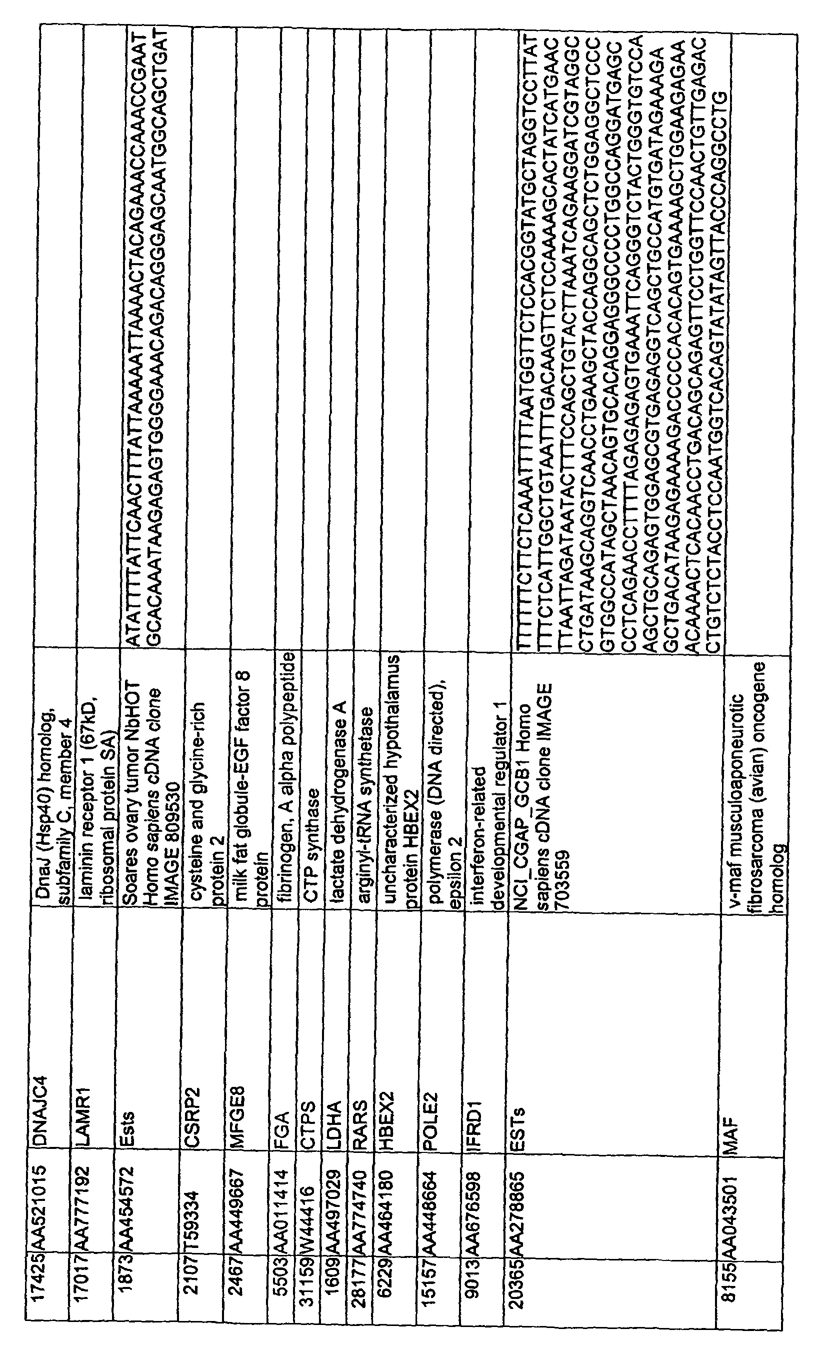

## Claims

1. A method for detecting and/or staging a malignant functional cell comprising measuring an expression profile of said malignant cell and a non-malignant cell, wherein said non-malignant cell and said malignant cell are capable of interacting.

2. A method according to claim 1, comprising determining the presence and/or amount of expression product of at least two reporter genes in a sample comprising said malignant functional cell and at least one non-malignant cell.

3. A method according to claim 1 or 2, wherein said reporter gene is involved with a proliferation/apoptosis pathway, a metabolism pathway, an immune competence pathway and/or a T cell infiltration pathway.

4. A method according to any one of claims 1-3, wherein said expression product comprises mRNA.

5. A method according to any one of claims 1-4, wherein said expression product comprises protein.

6. A method according to any one of claims 1-5, comprising determining in a sample comprising said non-malignant cell the presence and/or amount of expression product of at least 5 reporter genes listed in table 2.

7. A method according to any one of claims 1-6, comprising determining in a sample comprising said non-malignant cell the presence and/or amount of expression product of at least 10 reporter genes listed in table 2.

8. A method according to any one of claims 1-7, comprising determining in a sample comprising said non-malignant cell the presence and/or amount of expression product of at least 30 reporter genes listed in table 2.

9. A method according to any one of claims 1-8, comprising determining in a sample comprising said non-malignant cell the presence and/or amount of expression product of at least 50 reporter genes listed in table 2.

10. A method according to any one of claims 1-9, comprising determining in a sample comprising said non-malignant cell the presence and/or amount of expression product of each of the reporter genes listed in table 2.

11. A method according to any one of claims 1-10, wherein said malignant functional cell is capable of producing an antibody, enzyme and/or hormone.

12. A method according to any one of claims 1-11, wherein said malignant functional cell comprises a B cell.

13. A method according to any one of claims 1-12, wherein said malignant functional cell comprises a T cell.

14. A method according to any one of claims 1-13, wherein said malignant functional cell comprises a follicular lymphoma cell.

15. A method according to any one of claims 1-14, wherein said malignant functional cell comprises a mucosa-associated lymphoid tissue lymphoma cell.

16. A method according to any one of claims 1-15, wherein said malignant functional cell comprises a cell of a Grawitz tumour.

17. A method according to any one of claims 1-16, wherein said malignant functional cell comprises a prostate cancer cell.

18. A method according to any one of claims 1-17, wherein said non-malignant cell comprises a T cell.

19. A method according to any one of claims 1-18, wherein said non-malignant cell comprises a T helper cell.

20. An array comprising nucleic acid molecules capable of specifically binding to at least 5 reporter genes listed in table 2.

21. An array comprising nucleic acid molecules capable of specifically binding to at least 10 reporter genes listed in table 2.

22. An array comprising nucleic acid molecules capable of specifically binding to at least 30 reporter genes listed in table 2.

23. An array comprising nucleic acid molecules capable of specifically binding to at least 50 reporter genes listed in table 2.

24. An array comprising nucleic acid molecules capable of specifically binding to each of the reporter genes listed in table 2.

25. A method for detecting and/or staging a malignant functional cell comprising:
- providing an array according to any one of claims 20-24 with a sample comprising expression product from said malignant functional cell and from a non-malignant cell; and
- determining whether an expression profile indicative for the presence and/or stage of said malignant functional cell is obtained.

26. A method for treating a disease involving a malignant functional cell, comprising at least in part inhibiting an interaction between said malignant functional cell and a non-malignant cell.

27. A method according to claim 26, comprising at least in part inhibiting signaling of a T cell.

28. A compound capable of at least in part inhibiting signaling of a T cell for use as a medicament.

29. Use of a compound capable of at least in part inhibiting signaling of a T cell for the preparation of a medicament for treating a disease involving a malignant functional cell.

30. Use of a compound capable of at least in part inhibiting signaling of a T cell for the preparation of a medicament for treating follicular lymphoma.
